# EUROPEAN PATENT SPECIFICATION

(11) **EP 3 955 977 B1**
(45) Date of publication and mention of the grant of the patent: **19.08.2026**
(21) Application number: 20790996.1
(22) Date of filing: 17.04.2020
(51) Int. Cl.: A61L 27/18, A61L 27/50, A61L 27/52, A61L 27/56, A61F 2/24, D01F 6/62, D01D 5/00, D01D 5/08, B33Y 80/00, C12M 3/00, C12M 1/12, C12M 1/26, C12N 5/071

(54) **MEW TISSUE SCAFFOLD**
MEW-GEWEBEGERÜST
ÉCHAFAUDAGE DE TISSU PAR ÉLECTROIMPRESSION À CHAUD (MEW)

(30) Priority: 18.04.2019 AU 2019901344
(43) Date of publication of application: 23.02.2022
(73) Proprietor: University of Western Australia, Crawley, Western Australia 6009 (AU)
(72) Inventor: HUTMACHER, Dietmar, Kelvin Grove, Queensland 4059 (AU); JUAN PARDO, Maria Elena, Kelvin Grove, Queensland 4059 (AU); BAS, Onur, Kelvin Grove, Queensland 4059 (AU); TOOSISAIDY, Navid, Kelvin Grove, Queensland 4059 (AU); MELA, Petra, Kelvin Grove, Queensland 4059 (AU)
(74) Representative: Tiburzi, Andrea
(86) International application number: PCT/AU2020/050383
(87) International publication number: WO 2020/210877

(56) References cited:
- WO-A1-2014/158444
- US-A1- 2015 306 282
- HOCHLEITNER, G: "Advancing melt electospinning writing for fabrication of biomimetic structures", DISSERTATION ZUR ERLANGUNG DES NATURWISSENSCHAFTLICHEN DOKTORGRADES DER JULIUS-MAXIMILIANS-UNIVERSITAT WÜRZBURG [DISSERTATION TO OBTAIN THE SCIENTIFIC DOCTORAL DEGREE THE JULIUS MAXIMILIANS UNIVERSITY OF WÜRZBURG, 5 August 2018 (2018-08-05), XP055748932, Retrieved from the Internet <URL:https://opus.bibliothek.uni-wuerzburg.de/opus4-wuerzburg/frontdoor/deliver/index/docId/16219/file/Hochleitner_Gernot_Melt_Electrospinning_Writing.pdf> [retrieved on 20200610]
- ONUR BAS, D’ANGELLA DAVIDE, BALDWIN JEREMY G., CASTRO NATHAN J., WUNNER FELIX M., SAIDY NAVID T., KOLLMANNSBERGER STEFAN, REALI AL: "An Integrated Design, Material, and Fabrication Platform for Engineering Biomechanically and Biologically Functional Soft Tissues", ACS APPL. MATER. INTERFACES, vol. 9, 2017, pages 29430 - 29437, XP055748978
- JUNGST, T. ET AL.: "Melt electrospinning onto cylinders: effects of rotational velocity and collector diameter on morphology of tubular structures", POLYM. INT., vol. 64, 2015, pages 1086 - 1095, XP055204699, DOI: 10.1002/pi.4948
- MIGUEL CASTILHO, DRIES FEYEN, MARÍA FLANDES-IPARRAGUIRRE, GERNOT HOCHLEITNER, JÜRGEN GROLL, PIETER A. F. DOEVENDANS, TINA VERMONDE: "Melt Electrospinning Writing of Poly-Hydroxymethylglycolide- co-epsilon-Caprolactone-Based Scaffolds for Cardiac Tissue Engineering", ADV. HEALTHCARE MATER., vol. 6, no. 18, 2017, pages 1700311-1 - 1700311-9, XP055748980
- SACKS, M. S. ET AL.: "On the Biomechanics of Heart Valve Function", J. BIOMECH., vol. 42, no. 12, 2009, pages 1804 - 1824, XP026446836
- ANWARUL HASAN, KIM RAGAERT, WOJCIECH SWIESZKOWSKI, ŠEILA SELIMOVIĆ, ARGHYA PAUL, GULDEN CAMCI-UNAL, MOHAMMAD R.K. MOFRAD, ALI KHAD: "Biomechanical properties of native and tissue engineered heart valve constructs", J. BIOMECH., vol. 47, 2014, pages 1949 - 1963, XP055748984
- M. A. MEYERS, J. MCKITTRICK, P.-Y. CHEN: "Structural Biological Materials: Critical Mechanics-Materials Connections", SCIENCE, vol. 339, 2013, pages 773 - 779, XP055748987
- JETZE VISSER, FERRY P.W. MELCHELS, JUNE E. JEON, ERIK M. VAN BUSSEL, LAURA S. KIMPTON, HELEN M. BYRNE, WOUTER J.A. DHERT, PAUL D. : "Reinforcement of hydrogels using three-dimensionally printed microfibres", NAT. COMMUN., vol. 6, June 2015 (2015-06-01), pages 1 - 10, XP055748990

## Description

### Technical Field

This disclosure relates generally to soft tissue scaffolds used in tissue engineering, such as scaffolds for use as heart valve regeneration.

### Background

Valvular Heart Disease (VHD) is a significant health burden accountable for a third of cardiovascular disease resulting in more than 5.8 million deaths annually worldwide. The prevalence of VHD is expected to rise in developed countries due to increasing age of the population. For example, by 2020, about 20% of the European Union population will be over 65 years old. Additionally, valvular heart disease significantly affects children and young adults where statistically 8 out of 1000 births are affected by congenital valve disease and this is expected to triple by the year 2050 in developing countries. The main treatment method for diseased heart valves includes the surgical implantation of mechanical or biological prosthetic replacement valves. Although the replacement options perform an adequate job in enhancing quality of life for older patients, their application is often associated with several limitations and overall, the long-term survival rate ranges from 60 to 70 %.

Mechanical valves offer adequate durability within the native hemodynamic environment, but their design does not resemble the native valve geometry, thereby requiring anticoagulation therapy to diminish the possible risk of thromboembolism. On the other hand, biological prosthetics are decellularized valves derived from a porcine or ovine source roughly replicating the physiology of a human heart valve. Biological valves are considerably less thrombogenic, but they do not perform well under high pressure gradients and have a shorter life-span as they tend to degenerate leading to a life expectancy of only 10 - 15 years. The choice among the two different replacement valves may depend upon the pathology and age-group of the patient as each of these options is eve• more suited to a specific group of patients. For patients suffering from a congenital heart valve defect, the limitations associated with currently available replacement valves are amplified because of additional technical complications caused by smaller anatomical dimensions and imminent biological development. Specifically, the performance of biological and mechanical valves deteriorates at very small dimensions. Additionally, their inability to grow and remodel along with the somatic growth of the child necessitates multiple operations as the patient ages. Therefore, in the past 20 years there has been a growing amount of attention toward heart valve tissue engineering (HVTE) for congenital valve diseases. HVTE aims to overcome the disadvantages of current therapies by providing a biodegradable yet mechanically stable three-dimensional (3D) construct (scaffold) that is capable of guiding tissue growth, remodelling and repair before the body reabsorbs it, leaving behind a complete functional, regenerated endogenous heart valve. Despite the progress in HVTE, the current constructs still are unable to result in a regenerated endogenous heart valve.

Further relevant information regarding melt electrowriting scaffolds can be found-in Hochleitner, G., Advancing melt electrospinning writing for fabrication of biomimetic structures, Dissertation, Julius-Maximilians-Universität Würzburg, 2018, and Bas et al., An Integrated Design, Material, and Fabrication Platform for Engineering Biomechanically and Biologically Functional Soft Tissues, ACS Applied Materials & Interfaces, 9 (2017), 29430-29437.

### Summary

An embodiment provides a melt electrowritten anisotropic soft tissue scaffold, comprising:
a first set of fibres arranged approximately in parallel relative to one another, each fibre of the first set of fibres having a serpentine arrangement forming peaks and troughs, wherein adjacent peaks for each fibre of the first set of fibres are separated by a first distance; and
a second set of fibres arranged approximately in parallel relative to one another, the second set of fibres being arranged transversely relative to the first set of fibres where one or more fibres of the second set of fibres connect adjacent fibres from the first set of fibres, each fibre of the second set of fibres having a serpentine arrangement forming peaks and troughs;
wherein a pathlength of a fibre of the first set of fibres over the first distance is unequal to a pathlength of a fibre of the second set of fibres over a same distance as the first distance. The first set of fibres and the second set of fibres may be provided in a first region of the scaffold.

An embodiment provides a melt electrowritten soft tissue scaffold, comprising:
a body having a first region comprising a first set of fibres and a second set of fibres, the first region being anisotropic;
wherein the first set of fibres are arranged approximately parallel relative to one another, each fibre of the first set of fibres having a serpentine arrangement forming peaks and troughs, the first set of fibres have a first Young's modulus;
wherein the second set of fibres are arranged approximately parallel relative to one another, the second set of fibres being arranged transversely relative to the first set of fibres, each fibre of the second set of fibres having a serpentine arrangement forming peaks and troughs, the second set of fibres have a second Young's modulus; and
wherein the first Young's modulus is unequal to the second Young's modulus. The second Young's modulus may be at least double the Young's modulus of the first set of fibres.

A pathlength of a fibre of the first set of fibres over a predefined distance may be unequal to a pathlength of a fibre of the second set of fibres over the predefined distance.

By providing two sets of fibres with differing pathlengths, an anisotropic scaffold may be produced that can mimic the mechanical properties of native tissue. For example, the scaffold may provide a structural analogue to collagen structures. Such analogues may help to improve the ability to regenerate tissue, such as heart valve tissue.

The pathlength of a fibre of the first set of fibres over the first distance may be greater than the pathlength of a fibre of the second set of fibres over a same linear distance as the first distance. In some embodiments, increasing the pathlength of the fibre of the first set of fibres relative to the pathlength of the fibre of the second set of fibres may increase an anisotropic ratio of the first set of fibres to the second set of fibres. This means that when the first and second set of fibres are stretched to be elongate, the first set of fibres may be stretched further than the second set of fibres. This may help to provide a scaffold having two sets of fibres that are connected to one another, but the properties of the first and second set of fibres may be independent of one another. Adjacent fibres of the first set of fibres may be separated by a second distance. The second distance may be unequal to the first distance.

A region proximate to peaks of adjacent fibres of the first set of fibres may be connected to one or more fibres of the second set of fibres.

Another embodiment provides a melt electrowritten anisotropic soft tissue scaffold, comprising:
a first set of fibres arranged approximately in parallel relative to one another, each fibre of the first set of fibres having a serpentine arrangement forming peaks and troughs, wherein adjacent peaks for each fibre of the first set of fibres are separated by a first distance; and
a second set of fibres arranged approximately in parallel relative to one another, the second set of fibres being arranged transversely relative to the first set of fibres where one or more fibres of the second set of fibres connect adjacent fibres from the first set of fibres, each fibre of the second set of fibres having a serpentine arrangement forming peaks and troughs;
wherein each fibre of the first set of fibres is separated by a second distance, the second distance being unequal to the first distance. The first and second set of fibres may be provided in a first region of the scaffold.

The terms "transversely" and "transverse" as used herein is to be interpreted broadly to mean an angle formed between the first and second set of fibres ranges from about 1° to about 179°.

The first and/or second set of fibres may include a region having an elongate straight fibre arrangement. The straight fibre arrangement may be in addition to the serpentine arrangement. Put another way, the first and/or second set of fibres may include a region where one or more of the fibres are not serpentine.

The second set of fibres may be approximately 2-10 times stiffer than the first set of fibres. For example, the second set of fibres may be 8 times stiffer compared to the first set of fibres. Increasing the stiffness of the second set of fibres relative to the first set of fibres may be achieved by decreasing the pathlength of the fibres of the second set of fibres relative to the first set of fibres.

The first set of fibres of the disclosed scaffold may have a Young's modulus of approximately 0.1 MPa to 10 MPa. In an embodiment the first set of fibres may have a Young's modulus of about 1 MPa. The second set of fibres may have a Young's modulus of approximately 0.1 MPa to 10 MPa. In an embodiment the second set of fibres may have a Young's modulus of about 5 MPa. Increasing the first distance relative to the second distance may increase an anisotropic ratio of the first set of fibres to the second set of fibres. The anisotropic ratio being the ratio of the difference in mechanical properties of the first and second set of fibres. The degree of the anisotropy may be changed by tuning the design of the fibres and the resulting construct may have a Young's modulus of 0.1 MPa to 10 MPa in each loading direction. The first distance may be approximately 1-10 times larger than the second distance, such as 2-4 times larger. For example, the first distance may range from about 0.5 mm to about 2.5 mm, such as about 1.0 mm to about 2.0 mm. The second distance may range from about 0.1 mm to about 2.0 mm, such as about 0.25 mm to about 0.50 mm. This spacing means that a gap between adjacent fibres from the first set of fibres is about 0.1 mm to about 2.0 mm, such as about 0.1 mm to about 0.5 mm. It should be appreciated that local variations means that the spacing may be less or more than about 0.1 mm to about 2.0 mm. This may be especially true once the scaffold is seeded with cells and/or once implanted *in situ.* The first and second distance may be selected so that a spacing between adjacent fibres from the first set of fibres and/or the second set of fibres is such that a resulting pore size allows cellular proliferation. Therefore, a pore size of about 2.0 mm tends to be an upper limit for the pore size as pores larger than 2.0 mm tend to impede proliferation of cells through a scaffold and promote laminar rather than 3D tissue growth. As the pore size increases up to about 2.0 mm, it is a matter of time for the cells to become confluent and fill in all the pore spaces with both cells and extracellular matrix. However, it should be appreciated that some applications may require a pore size greater than 2.0 mm and the disclosure is not limited to a maximum pore size of 2.0 mm.

The first and second set of fibres may be arranged to form a first layered structure. In some embodiments the scaffold comprises more than one layer. A fibre orientation and design of each layer may be different. The first and second distance of each layer may be different.

In some embodiments of the disclosed scaffold the fibres of the first set of fibres may be interwoven with fibres of the second set of fibres. Alternatively, or in addition to, the fibres of the first and/or second set of fibres may be laminated one on top of another. Interwoven fibres may help to improve the connection between the first set of fibres and the second set of fibres. For example, the connection of the first set of fibres to the second set of fibres may be provided by fusion of the respective fibres. In some embodiments, a transition zone between the first set of fibres and the second set of fibres having a gradient in transverse angles is provided to avoid layer delamination of the first and second set of fibres. The first and second sets of fibres may be formed from a medical grade, biodegradable thermoplastic. The first and second set of fibres may be formed from different thermoplastics. The thermoplastic may be a homo-polymer or a co-polymer. In an embodiment the thermoplastic includes poly *ε*-caprolactone (PCL), a poly(glycolide-co-trimethylene carbonate-co-caprolactone) thermopolymer such as Strataprene^{®} from Poly-Med Inc, poly(carbonate urethane) urea, a poly urethane and/or poly(ester urethane)urea. The thermoplastic may be biodegradable. The thermoplastic may be non-biodegradable. The melt electrowriting conditions (temperature, pressure, etc.) are generally dependent on the type of thermoplastic used to form the scaffold. The fibres of the first and second set of fibres may have a diameter ranging from about 100 nm to about 100 µm. In some embodiments, the diameter is about 20 µm. The scaffold may further comprise a hydrogel. At least a portion of the first region may be embedded in the hydrogel.

The scaffold may comprise a planar region, such as a sheet e.g. a fabric. The scaffold may comprise a tubular region. A diameter of the tubular region may range from 0.5-50 mm. The tubular region may be a scaffold for regeneration of blood vessels and/or constructs for soft micro-actuators that represent a soft tissue in a robotic setup. The scaffold may form part of an actuator, for example a melt electrowritten scaffold for an actuator component. A combination of planar and tubular regions may be used. The scaffold may have 3D features, for example protrusions extending above a plane of a sheet or radially unsymmetrical portions. In some embodiments the scaffold is a heart valve leaflet scaffold. In these embodiments, the first set of fibres may be orientated generally in a radial direction of the heart valve leaflets and the second set of fibres may be orientated generally in a circumferential direction of the heart valve leaflets.

Another embodiment provides a melt electrowritten anisotropic soft tissue scaffold, comprising:
a first set of fibres having a first Young's modulus and a second set of fibres having a second Young's modulus, the first Young's modulus being unequal to the second Young's modulus;
wherein the first set of fibres are arranged transversely relative to the second set of fibres. The first and second set of fibres may be provided in a first region of the scaffold.

The first Young's modulus may be provided by the first set of fibres having a first degree of curvature and the second Young's modulus may be provided by the second set of fibres having a second degree of curvature. In some embodiments, straight fibres having specific mechanical properties and a resulting Young's modulus may be provided as the first and/or second set of fibres. Changing a fibre diameter, pore size, arrangement of a pattern of the first and/or second set of fibres (e.g., degree of curvature) in different loading directions may change the Young's modulus of the first and/or second set of fibres, which in turn may alter the anisotropic properties of the soft tissue scaffold.

An embodiment of the disclosed scaffold may further comprise a second region extending from the first region. The second region may support the first region, for example the second region may act as a support. The second region may be anisotropic or isotropic. The second region may be a soft tissue scaffold. The second region may be a mesh having fibres arranged in a first direction and a second direction. The first and second directions may be transverse to one another. A spacing between adjacent fibres in both the first and second directions may be the same. An embodiment may further comprise an intermediate region positioned at an interface of the first and second regions. The intermediate region may comprise a plurality of fibres. The intermediate region may reinforce the scaffold, for example to help withstand stresses applied to the scaffold once implanted and sutured to tissue.

The first region may be semicircular. The second region may extend from a curved side of the first region and a straight side of the first region forms an edge of the scaffold. In an embodiment the first region may comprise a plurality of semicircular regions where the vertices of adjacent semicircles are positioned proximate one another. The intermediate region may be positioned along the curved side. The intermediate region may comprise in an embodiment a first set of concentric semicircle fibres that are arranged parallel to one another, and a second set of fibres that connect adjacent concentric semicircle fibres. The first and second regions may be integral.

Another embodiment provides a method of producing an anisotropic soft tissue scaffold using melt electrowriting. The method comprises:
extruding a polymer melt through a nozzle to form a fibre;
depositing the fibre to form a first set of fibres that are arranged approximately parallel to one another, each fibre of the first set of fibres has a serpentine arrangement forming peaks and troughs, wherein adjacent peaks for each fibre of the first set of fibres are separated by a first distance; and
depositing the fibre to form a second set of fibres that are arranged approximately parallel to one another, the second set of fibres being transversely arranged relative to the first set of fibres where one or more fibres of the second set of fibres connect adjacent fibres from the first set of fibres, each fibre of the second set of fibres having a serpentine arrangement forming peaks and troughs. In an embodiment the first set of fibres are deposited so that the first set of fibres has a first Young's modulus and the second set of fibres are deposited so that the second set of fibres has a second Young's modulus. The first and second set of fibres may form a first region of the scaffold.

Another embodiment provides a method of producing an anisotropic soft tissue scaffold using melt electrowriting, the method comprising:
extruding a polymer melt through a nozzle to form a fibre;
depositing the fibre to form a body having a first region that is anisotropic, the first region comprising:
   a first set of fibres that are arranged approximately parallel to one another, each fibre of the first set of fibres has a serpentine arrangement forming peaks and troughs; and
   a second set of fibres that are arranged approximately parallel relative to one another, the second set of fibres being arranged transversely relative to the first set of fibres, each fibre of the second set of fibres having a serpentine arrangement forming peaks and troughs;
   wherein the first set of fibres are deposited so that the first set of fibres has a first Young's modulus and the second set of fibres are deposited so that the second set of fibres has a second Young's modulus.

A pathlength of a fibre of the first set of fibres over the first distance may be unequal to a pathlength of a fibre of the second set of fibres over a same distance as the first distance. The first set of fibres may be deposited so that adjacent peaks for each fibre of the first set of fibres are separated by a first distance. The first set of fibres may be deposited so that adjacent fibres of the first set of fibres are separated by a second distance. The first and second set of fibres may be deposited so that fibres of the first set of fibres are interwoven with fibres of the second set of fibres. The first and second set of fibres may be deposited so that a portion of the first set of fibres is fused to a portion of the second set of fibres. Fusion of the respective fibres may be carried out by depositing the fibre at a temperature above its melting point. For example, when the fibre is a PCL fibre, it may be deposited at a temperature above about 70 °C. The method may further comprise annealing the scaffold to improve the fusion of the respective fibres.

The method may comprise depositing a plurality of fibre layers to form a layered structure. The first and second set of fibres may be deposited so that they form a layered structure. The method may further comprise depositing a second or more layered structure. The layered structure may be deposited so that each layered structure has a different anisotropic direction. Put another way, the first set of fibres of each layered structure may be arranged to be transverse to one another.

An embodiment may further comprise depositing the fibre to form a second region extending from the first region. The second region may be isotropic. The second region may comprise a mesh having fibres arranged in a first direction and a second direction. The first and second directions may be transverse to one another. In an embodiment the first and second directions are perpendicular to one another. A spacing between adjacent fibres in both the first and second directions may be the same. An embodiment may further comprise depositing the fibre to form an intermediate region positioned at an interface of the first and second regions. The intermediate region may comprise a plurality of fibres. An embodiment may further comprise treating a surface of the scaffold to increase a hydrophilicity of the scaffold. The method may further comprise forming a hydrogel that at least partially embeds the first region.

The first and second set of fibres may be deposited onto a stage. The stage may be planar, tubular and/or a mould having 3D features. Therefore, the method may be used to prepare planar, tubular and/or scaffolds having 3D features. The method may further comprise depositing the first set of fibres generally in a radial direction and depositing the second set of fibres in a circumferential direction. The scaffold may be a heart valve leaflet scaffold.

Another embodiment provides a scaffold formed using the method as set forth above.

Another embodiment provides a method of melt electrowriting to form a soft tissue scaffold, comprising:
rotating a conductive mandrel around a longitudinal axis of the mandrel, the mandrel having a portion that is radially unsymmetrical;
extruding a polymer from a nozzle to form a fibre; and
depositing the fibre onto the mandrel at a winding angle relative to the longitudinal axis as the mandrel is rotating to form the scaffold.

By radially unsymmetrical, it is meant that a radius of the mandrel is not constant relative to the longitudinal axis and there may be more than one radially expending feature giving rise to different radii. By radially extending, it is meant in a direction extending away from a central axis of the mandrel and/or in a direction extending towards the central axis. **In** this way, radially extending includes features such as protrusions extending away from the central axis, and grooves and channels extending towards the central axis. The channels may be formed by the protrusions.

Melt electrowriting has typically only been able to provide flat and/or symmetrical structures, and typically non-soft tissue scaffolds such as bone scaffolds. By having a radially unsymmetrical portion of the mandrel, this may allow a scaffold to be formed using melt electrowriting that has 3D features that resemble native tissue, such as the sinuses of Valsalva.

Because of the ability of melt electrowriting to use medical grade plastics, providing a mandrel with a radially unsymmetrical portion may allow melt electrowriting to produce 3D patient-specific scaffold structures more easily and more cheaply compared to other methods used to form 3D scaffolds.

The step of depositing the fibres may form a first region of the scaffold. Depositing the fibre may include printing and/or winding the fibre onto the mandrel. The method may further comprise moving the nozzle and mandrel relative to one another. The mandrel may be moved laterally with respect to the nozzle. The mandrel may be moved longitudinally with respect to the nozzle. The nozzle may be moved in a perpendicular direction relative to a plane in which the mandrel laterally moves. Moreover, in some embodiments, the nozzle and mandrel may be moved relative to one another in more than three degrees of freedom, such as six degrees of freedom. **In** some embodiments, the nozzle and mandrel may be moved relative to one another in two, three, four, five or six degrees of freedom. By increasing the number of the degree of freedom movement of the nozzle to the stage(s) (e.g. mandrel) used in the method, complex printing patterns on the scaffolds may be achieved. This may also be important to ensure the consistency and accuracy of the printing as the position of the scaffold and the printing head (e.g. nozzle) can dynamically be adjusted to maintain the stability of the electrical field.

The method may further comprise varying the winding angle by adjusting a speed at which the mandrel and nozzle are moved relative to one another. The method may also comprise varying the winding angle by adjusting a mandrel rotation speed. The mandrel may be moved relative to the nozzle at a speed such that a translational speed of an outer surface of the mandrel moves at in a range from about 10 mm/min to about 2000 mm/min, such as about 1000 mm/min. The actual revolutions per minute of a mandrel at a given translational speed will depend on the radius of the outer surface of the mandrel. The method may further include varying a fibre spacing between adjacent fibres depositing onto the mandrel by controlling the rotation and/or relative movement of the mandrel and the nozzle. The fibre may be deposited onto the mandrel at one or more winding angles. The one or more angles may range from about 0-90°, such as 30-60°.

The fibre may be deposited onto the mandrel in one or more layers. Each layer may form a structure. More than one structure may be deposited onto the mandrel. The fibre of a first layer may be deposited onto the mandrel at a first temperature. The fibre of a second or more layers may be deposited onto the mandrel at a second temperature. The first temperature may be lower than the second temperature. The difference in temperature may help to fuse the different layers together. The fibre of each layer may be deposited onto the mandrel at a different winding angle. For example, one layer may have fibres deposited onto the mandrel at 30° and another layer may have fibres deposited onto the mandrel at 45°.

The method may further comprise providing a first component of the scaffold, then forming a second component of the scaffold over an outer surface of the first component. The first component may be formed using melt electrowriting. The first component may be formed on the mandrel.

The mandrel may comprise a first segment having a first formation and a second segment having a second formation. The first and second segments may be engaged with one another so that the second formation sleeves a portion of the first formation. The first component may be formed by depositing the fibre onto the first formation. The second component may be then formed by depositing the fibre onto at least the second formation.

The method may be solvent free. For example, the polymer may be extruded from the nozzle without the need for solvents. In this case, the extruded polymer may be a melt. The polymer may be those certified for implantation. The polymer may be a medical grade polymer. The polymer may be poly-*ε*-caprolactone (PCL). The fibre may be a PCL fibre.

The method may further comprise a step of post-functionalising the scaffold. Post-functionalisation may include surface activation by plasma and/or embedding the scaffold within a hydrogel to form a fibre-reinforced hydrogel. The hydrogel may be biologically degradable. The hydrogel may be biologically non-degradable. Post-functionalisation may be carried out after the scaffold has formed and before the scaffold is removed from the mandrel. Therefore, post-functionalisation may occur when the scaffold portion is on the mandrel.

Another embodiment provides a soft tissue scaffold formed using the method as set forth above.

Another embodiment provides a melt electrowritten soft tissue scaffold, comprising:
a first hollow segment that is radially symmetrical and having a longitudinal axis;
a second hollow segment that is radially unsymmetrical and associated with the first hollow segment
wherein the first and second segments are formed from a fibre that is orientated relative to the longitudinal axis at one or more angles.

The first segment may have fibres arranged relative to the longitudinal axis at a first angle. The second segment may have fibres arranged relative to the longitudinal axis at a second angle. The scaffold may further comprise two or more layers. Each layer may have an average fibre angle, diameter and distance that is different from one another. The one or more angles may range from about 0-90°, such as 30-60°. A plurality of layers may form a structure. The scaffold may have more than one structure. The more than one structure may be arranged radially and/or longitudinally relative to one another.

The fibre may have a diameter ranging from about 10 nm to about 100 µm. A spacing between adjacent fibres may form pores. Therefore, in some embodiments, the scaffold may comprise pores. Diameters of adjacent fibres and the spacing between adjacent fibres may determine the pore size. The pores may have a size ranging from about 1 µm to about 5 mm, for example about 10 µm to about 100 µm. The pores may help to allow cellular growth in and around the scaffold. Therefore, the size of the pores may be determined by the cells intended to be seeded onto the scaffold and the type of tissue that is intended to be grown on the scaffold.

The scaffold may have mechanical properties that resemble a native tissue that the scaffold intends to regenerate. For example, the scaffold may have mechanical properties that resemble soft tissue, such as a native aortic root. The scaffold may have mechanical properties such that when infused with cellular material, such as epithelial cells capable of forming an aortic root, the infused scaffold has mechanical properties similar to native tissue. It should be appreciated that the mechanical properties of a fresh scaffold i.e. one that has not yet been implanted into a patient will change over time once the scaffold degrades *in situ.* The rate of scaffold degradation will be determined by the polymer(s) used to form the fibre, the patient, the type(s) of tissue to be formed on the scaffold, and the forces exerted onto the scaffold and/or regenerated tissue *in situ.*

The scaffold may be a scaffold for an aortic root. The second segment may comprise bulges extending in a radial direction forming a scaffold for sinuses of Valsalva. The scaffold may further comprise a leaflet scaffold portion arranged within a cavity formed by the bulges. The leaflet scaffold position may be used as a scaffold for a valve of the aortic root.

The scaffold may further comprise a hydrogel. The scaffold may be embedded in the scaffold or the hydrogel may be embedded in the scaffold. The hydrogel may be used as a mode of cell delivery on to the scaffold where the combination hydrogel and scaffold provides an optimal cell-scaffold interaction and mechanical integrity respectively.

The scaffold may have a diameter ranging from about 1mm to about 50 mm at the aortic wall. The fibre may be made of a polymer, co-polymer, or composite, e.g. aliphatic polyesters/polyethers including, and may include PCL, PLLA, PLGA, PDO, PMMA.

Another embodiment provides a melt electrowriting system for forming a soft tissue scaffold, comprising:
a stage;
a conductive mandrel configured to be secured to the stage in use, the mandrel having a longitudinal axis and a portion that is radially unsymmetrical, wherein the conductive mandrel is rotatable around the longitudinal axis;
a nozzle for extruding a polymer fibre; and
a power supply for applying a potential across the nozzle and conductive mandrel.

The mandrel may be formed from one or more metals such as aluminium, stainless steel, copper. Alternatively, or in addition to, the mandrel may be formed from a conductive polymer. The mandrel may be formed from a non-conductive material covered with a conductive material. The mandrel may have a conductive core, such as a metal rod. In an embodiment, the mandrel is a conductive plastic, such as conductive poly(lactic acid) having a metal core. The metal core may act as a shaft.

The mandrel may be formed from one or more segments that are engageable with one another. The mandrel may comprise a first segment engageable with a second segment. The first segment may have a first formation and the second segment may have a second formation. The first formation may sleeve a portion of the second formation when the first and second segments are engaged with one another.

The stage and/or nozzle are moveable relative to one another. The stage and nozzle may be moveable in an X, Y and Z direction relative to one another. The degree of the freedom of the stage can be increased to facilitate more complex movements. For example, the stage and nozzle may be moveable relative to one another in more than one degree of freedom, such as three or more degrees of freedom, for example six degrees of freedom.

Another embodiment provides a scaffold prepared using the system as set forth above. The scaffold may be as set forth above.

### Brief Description of Figures

Embodiments will now be described by way of example only with reference to the accompanying non-limiting Figure.
Figure 1 shows an embodiment of a scaffold architecture.
Figure 2 shows another embodiment of a scaffold architecture.
Figure 3 shows another embodiment of a scaffold architecture.
Figure 4 shows SEM images of embodiments of PCL melt electro-spun scaffolds; a) 20 layers, straight fibres, 0.5 circumferential & 2mm radial pore-size; b) 20 layers, helical patterns, 0.5mm circumferential & 2mm radial pore size; c) 20 layers, helical patterns, 0.25mm circumferential & 2mm radial pore size; d) Fibre stacking across the layers; and e) Fusion of circumferential and radial fibres.
Figure 5 shows a schematic illustration of an aortic valve leaflet collagen fibre deformation behaviour and the cause of J shaped stress/strain curvature.

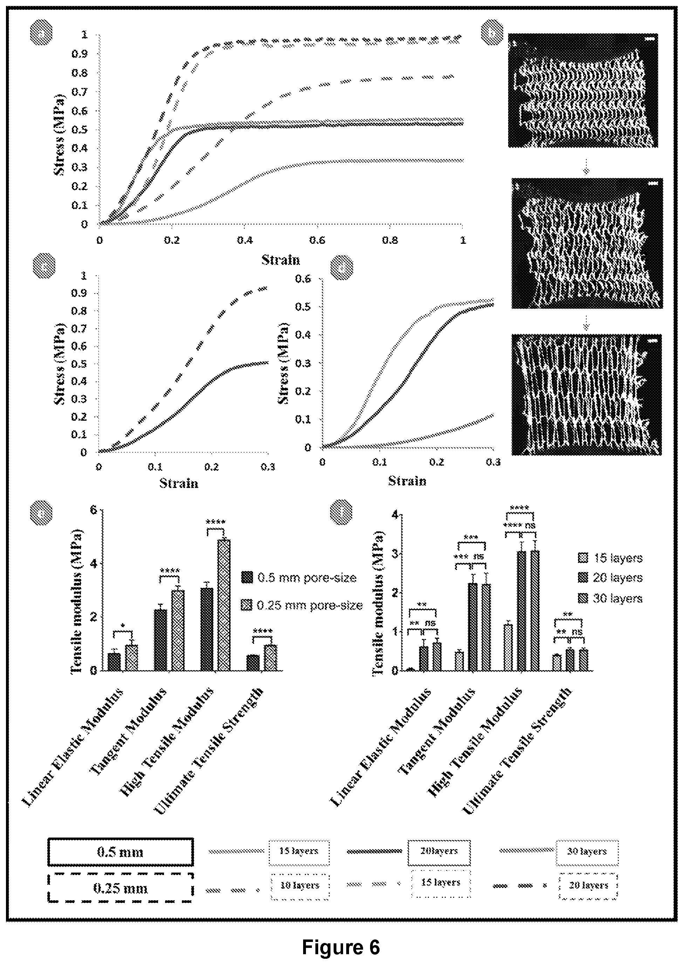

Figure 6 shows mechanical characterization of melt electro-written scaffolds by uniaxial tensile testing in the circumferential direction: a) Representative stress/strain curves of scaffolds with varying pore-size and layer number up to a 100% strain; b) Sequential recruitment of fibres from serpentine to a straight architecture (scale bar=2mm); c) Representative stress/strain curves of scaffolds with 20 layers, 0.5 and 0.25 pore-sizes at 30% strain; d) Representative stress/strain curves of scaffolds with 15, 20 and 30 layers and 0.5mm pore-size; e) Tensile modulus of scaffolds with variant pore-size; and f) tensile modulus of scaffolds with variant layer number.
Figure 7 shows uniaxial tensile tests performed to characterize the effect of curvature degree on strain at maximum stress: a) representative stress/strain curves; and b) tensile modulus at different regions of the J shaped curve.
Figure 8 shows anisotropic properties of the optimal scaffold and its comparison with the native aortic valve leaflet: a) representative stress/strain curve in circumferential (0.25mm pore) and radial (1 & 2mm pore); b) anisotropic ratio of MEW scaffold; and c) high elastic modulus of the PCL MEW scaffold compared with the porcine, ovine and human aortic valve leaflet in circumferential and radial test directions (native tissue properties are represented by dashed and solid lines represents values for radial and circumferential direction respectively^{]})
Figure 9 shows stress relaxation response of scaffold characterized with a uniaxial tensile testing setup.
Figure 10 shows experimental and predicted fatigue properties of an embodiment of a scaffold tested in a) circumferential direction b) radial direction.
Figure 11 shows characterization of hysteresis properties of an embodiment of a scaffold: a) representative curve in circumferential direction; b) radial direction; and c) the effect of strain at unloading/ loading ratio.
Figure 12 shows: a) gross appearance; b) SEM images; and c) live/dead staining of an embodiment of a scaffold of the disclosure-human/HUVSMC (Human Umbilical Vein Smooth Muscle Cells) encapsulated fibrin composite after static cultivation for 1 and 2 weeks.
Figure 13 shows immunohistochemical analysis of an embodiment of a scaffold of the disclosure, the scaffold being human/HUVSMC (Human Umbilical Vein Smooth Muscle Cells) encapsulated fibrin composite after static cultivation for 1 and 2 weeks: staining for collagen type I (i,v) (green) and collagen type III (iii, vi) (red), revealed collagen synthesis during static cultivation. The majority of the seeded cells stained positive for α-SMA (ii, vi). Scale bars (b): 500 µm; (c): 200 µm; (d): i, ii, iii, v, vi, vii: 100 µm and iv, viii: 200 µm.
Figure 14 shows a silicone aortic root analogue having sutured thereto three single leaflet valve scaffolds of an embodiment of the disclosure: (i) sideview highlighting the suturing path; (ii) aortic view; and (iii) ventricular view.
Figure 15 shows an opening and closing sequence of the valve of Figure 14. Scale bar: 5mm
Figure 16 shows a graph plotting the performance of the valve of Figure 14 under physiological aortic pressure and flow conditions.
Figures 17a-c shows various embodiments of scaffold architectures.
Figure 18 shows an embodiment of a heart valve scaffold having two regions.
Figure 19 shows a schematic representation of an embodiment of a scaffold having two regions.
Figure 20 shows a schematic representation of another embodiment of a scaffold having two regions and an intermediate region.
Figure 21 shows an embodiment of a tubular heart valve scaffold having two regions.
Figure 22 shows (a) a graph plotting the performance of the valve of Figure 21 under physiological aortic and pulmonary pressure and flow conditions, and (b) the various stages of valve opening and closing during the aortic pressure and flow conditions of Figure 22a.
Figure 23 shows an embodiment of a melt electrowriting system.
Figure 24 shows (a) a side view (b) top view and (c) perspective side view of an embodiment of mandrel used in the system of Figure 23.
Figure 25 shows an embodiment of two-part mandrel used in the system of Figure 22.
Figure 26a shows an embodiment of mandrel used in the system of Figure 23.
Figure 26b shows an embodiment of a portion of a scaffold laid over a segment of the mandrel from Figure 26a.
Figure 26c shows an embodiment of a scaffold prepared using the mandrel of Figure 25a.
Figure 27 shows embodiments of scaffolds prepared with different winding fibre angles.
Figure 28 shows microscopic images of tubular scaffolds with different winding angles.
Figure 29 shows a multi-layer structure soft tissue scaffold.
Figure 30 shows different dimensioned multi-layer structure soft tissue scaffolds.
Figure 31 shows winding angle and fibre diameter characterization of tubular Melt Electrowriting (MEW) scaffolds: (a) winding angle over wall and sinuses; (b) fibre diameter over wall and sinuses; and (c) a statistical comparison of winding angle and fibre diameter between wall and sinuses.
Figure 32 shows the viability of HUVSMCs seeded directly onto MEW scaffolds with 0.5 mm straight, 0.5 mm serpentine and 0.25 mm serpentine pore sizes, and cultured under static conditions for 1 and 2 weeks: a) gross appearance; b) SEM images; and c) live/dead staining. Scale bars a: 2mm; b: 500 µm; c: 200 µm.

### Detailed Description of Embodiments

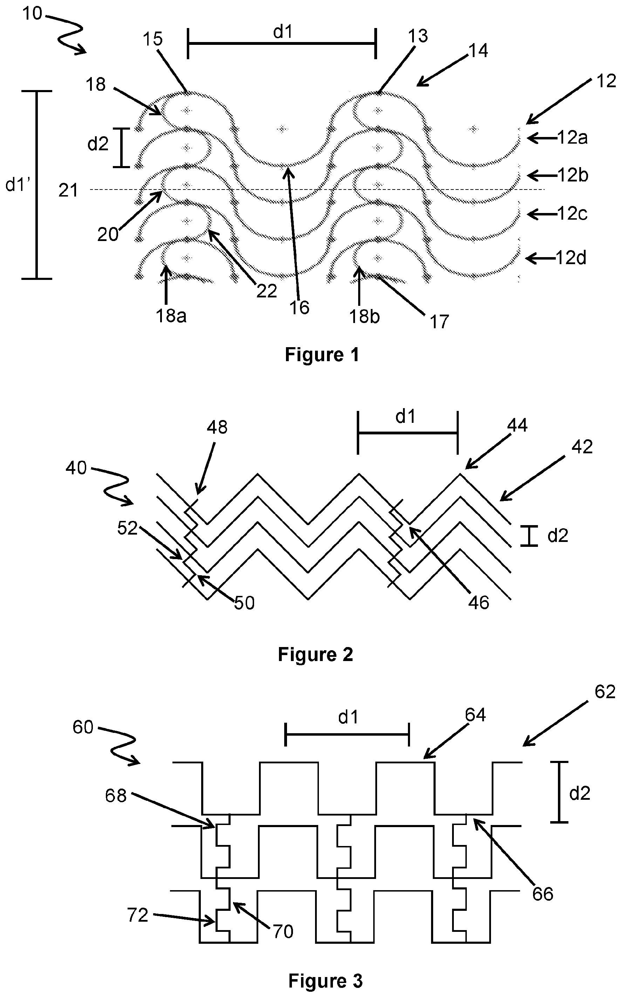

Figure 1 shows an embodiment of a melt electrowritten anisotropic soft tissue scaffold. The scaffold in Figure 1 is in the form of a sheet 10. The sheet 10 has a first set of fibres 12. The first of fibres 12 is made from a plurality of fibres (12a, 12b ... 12x) that are arranged approximately parallel to one another. Each fibre 12a-x has a serpentine arrangement, such as a meandering non-linear arrangement relative to a longitudinal direction, as represented by dashed line 21, of the first set of fibres 12, having peaks in the form of upper portion 14 and troughs in the form of lower portion 16. Each upper portion 14 of each fibre 12a-x is separated by a first distance d1. The first distance d1 is common for a spacing of adjacent apexes of all upper portions peaks for the fibres 12a-x of the first set of fibres 12. In the embodiment of Figure 1, each fibre 12a-x has a generally sinusoidal waveform, so the spacing between upper portions 12 is approximately the same as the spacing between the lower portions 16. Adjacent fibres (e.g. 12a and 12b) of the first set of fibres are separated by a second distance d2. In the embodiment of Figure 1, the first set of fibres 12 are provided as semi circles with a diameter of about 0.5 mm where d1 is larger than the diameter. This helps to control and mimic the anisotropy of the sheet.

The sheet 10 has a second set of fibres 18 arranged approximately transversely to the first set of fibres 12. The term "transversely" is to be interpreted broadly to mean the first set of fibres 12 and the second set of fibres 18 are arranged at an angle relative to one another, such as between 0°-90° e.g. approximately 30°-90°. Similar to the first set of fibres 12, the second set of fibres 18 are made up from a plurality of fibres (18a-x), with each fibre having a peak in the form of left portion 20 and trough in the form of right portion 22. The second set of fibres have a generally sinusoidal waveform. The second set of fibres 18 are connected to the first set of fibres 12. Figure 1 shows the connection point e.g. 13 between the first and second set of fibres as being at the peaks 14 of the first set of fibres 12 and an inflection point between the left portion 20 and right portion 22 of the second set of fibres 18. Put another way, the second set of fibres 18 are connected to a region proximate the upper portions 14 of the first set of fibres 12. In some embodiments the second set of fibres 18 are attached at locations other than or in addition to the upper portions 14 such as proximate or remote from the upper portion 14 and/or left portion 20 or right portion 22.

It should be appreciated that the term "peak", "trough", "upper portion", "lower portion", "left portion" and "right portion" are relative terms and do not limit the sheet 10 to any particular orientation. Put another way, each fibre has a longitudinal direction (i.e. 21), where a pathlength of the fibre is positioned in an alternating fashion on either side of the longitudinal direction in a left-right or up-down manner to provide a meandering fibre path. As an example, a top-to-bottom inversion of the sheet 10 would convert peaks 14 to trough 16, and vice versa, and a left-to-right inversion of the sheet 10 would convert left portions 20 to right portions 22.

A pathlength of the first set of fibres 12 for the first distance d1 is unequal to a pathlength of a fibre of the second set of fibres between a distance d1^{'} that is the same as the first distance d1. In the embodiment of Figure 1, the pathlength for the first set of fibres 12 is larger than the pathlength for the second set of fibres 18. The pathlength is the total length of the fibre for the first distance when the fibre is stretched longitudinally e.g. a total length of the fibre 12a between point 13 and 15. This higher pathlength for the first set of fibres 12 is partially attributed to the first set of fibres 12 having a higher degree of curvature compared to the second set of fibres 18. A greater pathlength allows the first set of fibres 12 to be stretched further from the serpentine orientation of Figure 1 to a straight orientation allowing for a large extension at a low applied stress/strain compared to the second set of fibres 18. Once the first set of fibres 12 and/or the second set of fibres 18 are stretched to their straight (i.e. elongate) orientation, a transition from the initial linear low stress/strain relationship to a high (steep) stress/strain relationship occurs up until reaching a constant ultimate tensile stress. Therefore, by providing a higher degree of curvature, the sheet 10 can be stretched further in a general direction of the first set of fibres 12 compared to the second set of fibres 18 before the transitions from the low stress/strain to high stress/strain. Put another way, the sheet 10 has different stretching characteristics (i.e. different mechanical properties) in the X and Y direction.

Increasing the first distance d1 relative the second distance d2, assuming the pathlength of the first set of fibres 12 for the first distance d1 is greater than the pathlength of the second set of fibres 18, an anisotropic ratio of the first set of fibres 12 relative the second set of fibres 18 can also be increased. The anisotropic ratio is a measure of the stretch of the sheet 10 in a direction of the first fibres 12 to the stretch of the sheet 10 in a direction of the second set of fibres 18. Put another way, the first set of fibres 12 can be stretched further than the second set of fibres 18 before reaching a constant ultimate tensile stress. In the embodiment of Figure 1, the first set of fibres 12 have a high tensile modulus of approximately 1 MPa and the second set of fibres 18 have a high tensile modulus of approximately 5 MPa. Increasing the number of fibres that make up the first and/or second set of fibres will increase the ultimate tensile stress of the set of fibres. For example, if the second distance d2 is decreased but the first distance d1 remains the same (i.e. the density of the first set of fibres is increased), an ultimate tensile stress of the sheet 10 will increase in a direction of the first fibres 12 but the large extension at a low applied stress/strain will remain the same. Increasing the density of the second set of fibres 18 relative to the first set of fibres 12 will increase the ultimate tensile stress of the second set of fibres 18. This means that the specific mechanical properties of the sheet 10 can be adjusted by changing the first distance d1, the second distance d2, the pathlength of the first and second set of fibres, and the density of the first and/or second set of fibres. However, by keeping the pathlength of the first and second set of fibres different, the sheet will be anisotropic since the sheet 10 will have different mechanical properties in different directions i.e. along a direction of the first and second fibres.

The first distance d1 in the embodiment of Figure 1 ranges from about 0.5 mm to about 2.5 mm, such as about 1.0-2.0 mm. The second distance d2 ranges from about 0.1 mm to about 0.5 mm. Decreasing d2 helps to increase the ultimate tensile strength of the first set of fibres. Because the sheet 10 acts as a soft tissue scaffold, the size of the pores formed between adjacent fibres can be important. If the pore size is too small, then this will prevent cellular infiltration into the scaffold. Cellular adhesion may also be affected. If the pore size of too large, then correct cellular infiltration and growth may be diminished. For example, if the pore size is too large, cells will first attach to a perimeter of the pore then grow radially inwards, but radially inwards growth can only continue if the cells are adequately supported.

Therefore, a size of the pores formed by the first and second fibres should be about 1 µm to about 400 µm.

It should be appreciated that not all 3D printing devices such as melt electrowriting apparatus can provide a sheet with such fine details as the resolution of the fibres is often limited to about 200 µm. Such large fibres would not be able to provide a soft tissue scaffold having the anisotropic characteristics and that can support cellular growth. In some embodiments, a diameter of the first and second set of fibres ranges from about 100 nm to about 100 µm, such as about 20 µm. In some embodiments, the fibre comprises PCL. In some embodiments, the fibre is a PCL fibre. Other polymers which can be processed by melt electrowriting can also be used to form the fibres.

Providing a scaffold with anisotropic mechanical properties can help to provide structural analogues to collagen structures. This means that a soft tissue scaffold having analogous mechanical properties to native tissue can be used to regenerate damaged and/or diseased tissue. For example, heart valve leaflets can be stretched further in a radial direction compared to a circumferential direction. Therefore, a soft tissue scaffold with anisotropic mechanical properties may be useful as a scaffold for regenerating heart valve leaflets. In some embodiments, the first set of fibres 12 (with the higher degree of curvature) would be orientated generally in a radial direction and the second set of fibres 18 (with a lower degree of curvature) would be orientated generally in the circumferential direction, providing a heart valve leaflet structural that is analogous to a native collagen structure.

Figure 1 shows an embodiment where the first set of fibres 12 and second set of fibres 18 have a generally sinusoidal waveform. Some embodiments may have fibres with a straight (i.e. elongate) regions, square wave form and/or zig zag waveform. A combination of fibre orientations is used in some embodiments. For example, the first set of fibres may have a serpentine arrangement and the second set of fibres may have a square waveform. In the embodiment of Figure 2, the sheet 40 has a first set of serpentine fibres 42 orientated to have a zig-zag orientation. Each fibre of the first set of fibres 21 has a peak in the form of upper peak 44 and a trough in the form of lower peak 46. Adjacent upper peaks 44 are separated by a first distance d1. Each fibre of the first set of fibres 42 are separated by a second distance d2. The sheet 40 also has a second set of fibres 48, where each fibre of the second set of fibres has a zig-zag orientation having a peak in the form of left peak 52 and a trough in the form of right peak 50. The degree of curvature of the first set of fibres 42 is greater than the degree of curvature of the second set of fibres 48.

Figure 3 shows an embodiment of a sheet 60 having a first set of serpentine fibres 62 having a square waveform arrangement having a peak in the form of upper section 64 and a trough in the form of lower section 66. Central regions of adjacent upper sections 64 are separated by a first distance d1. Each fibre of the first set of fibres is arranged approximately parallel to one another and is separated by a second distance d2. A second set of fibres 68 having a square waveform arrangement having peaks in the form of right section 70 and troughs in the forms of left sections 72. The first set of fibres 62 has a higher degree of curvature than the second set of fibres 68.

The term "serpentine" is to be interpreted broadly to mean a fibre that meanders in an alternating fashion on either side about a longitudinal direction. For example, in the embodiment of Figure 1, the longitudinal direction of the first set of fibres 12 is represented by dashed line 21, and each fibre of the first set of fibre meanders in an alternate manner about the longitudinal direction 74 to form the upper sections 64 and lower sections 66.

A plurality of first and/or second set of fibres in some embodiments are stacked on top of one another. For example, the first set of fibres 12 can have 10-30 layers of fibres forming a layered structure. In some embodiments, up to 2500 layers of the first and/or second set of fibres are stacked on top of one another. In some embodiments, the number of layers ranges from 1 to 2500. A single layer has a thickness approximately the same as the diameter of the fibre. 2500 layers can have a thickness (extending in the Z direction) of up to about 10 cm. In some embodiments, a plurality of sheets are combined to form the soft tissue scaffold. Each plurality of sheets can be a layered structure. In these embodiments, each sheet can be the same, or a combination of different sheets can be used, for example a two-sheet scaffold having sheet 10 and sheet 60. A longitudinal direction of the first set of fibres for each sheet can be arranged parallel to one another and/or transverse relative one another. In some embodiments, adjusting the angle of the longitudinal direction of the first set of fibres relative to one another for each sheet helps to control the anisotropic behaviour of the resulting scaffold. When the scaffold has a plurality of layers, the individual fibres from each layer can be stacked so that the resulting multi-layer scaffold has walls or similar extending from an outer to an inner layer (i.e. in a Z direction) that have a serpentine arrangement. This means that in addition to having different mechanical properties in the X/Y direction, the soft tissue scaffold can have different mechanical properties in the Z direction.

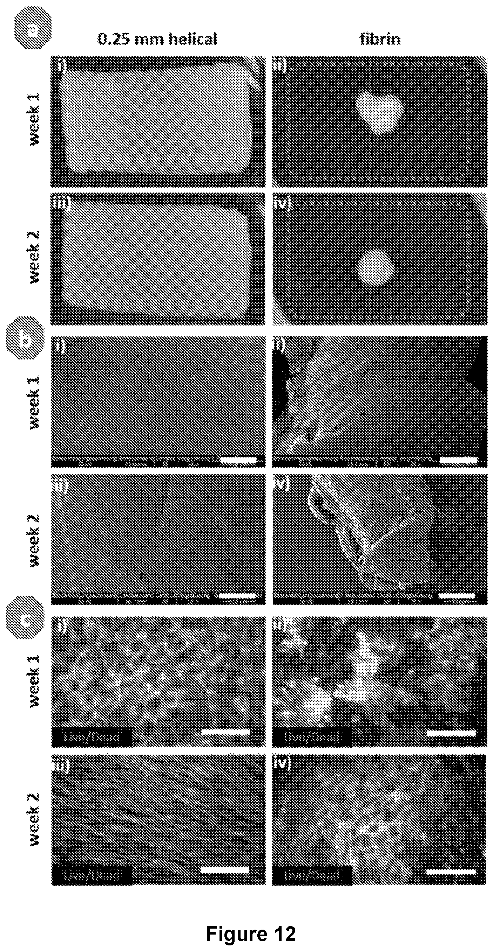

To form the sheet 10, a Melt Electrowriting (MEW) apparatus and/or system is used to melt a polymer and extrude it through a nozzle to form a fibre. An embodiment of a MEW apparatus is shown in Figure 12. The fibre is deposited onto a stage by applying a potential between the nozzle and the stage. A plurality of fibres are deposited approximately parallel to one another to form the first set of fibres 12. The second set of fibres 18 is also formed by depositing a plurality of approximately parallel fibres at an angle transverse to the first set of fibres 12. In some embodiments, the first and second set of fibres 12/18 are deposited so that fibres of the first set of fibres 12 are interwoven with fibres of the second set of fibres 18. Such an arrangement helps to improve the bonding of the first set of fibres 12 and the second set of fibres 18 at the contact points e.g. 13. If the temperature of the fibre being deposited is sufficiently high enough, it will fuse to an already deposited fibre, forming a contact point. To help ensure the temperature of the fibre being deposited it high enough, a temperature of the nozzle in some embodiments is higher than a temperature used to form the polymer melt prior to extrusion. For example, in some embodiments the nozzle is at about 85 °C and the melt is at about 75 °C when PCL is used to form the fibre.

The first and second set of fibres in some embodiments are deposited to form a layered structure. In these embodiments, the method can further comprise depositing a second or more layered structure e.g. a plurality of layered structures. Each layered structure can be formed by depositing a plurality of first and/or second set of fibres one on top of another. A longitudinal direction of the first set of fibres in one layer can be arranged parallel and/or at an angle to a longitudinal direction of the first set of fibres in the second or more layers.

The shape of the stage will determine to some extent the shape of the sheet 10. For example, a planar stage will generally result in a planar scaffold. However, if a tubular stage, such as a mandrel, is used, the scaffold will take a tubular form. Therefore, the scaffold can take the form of many different shapes. For example, a scaffold for a blood vessel can have a polymer architecture as depicted in Figure 1. The stage in some embodiments also includes 3D features that will give rise to a scaffold having the same 3D features. For example, the stage can have elements that form a mould to form leaflets for an aortic root. In these embodiments, the first set of fibres 12 would be deposited in a radial direction to reflect the mechanical properties of the leaflets in the radial direction and the second set of fibres 18 are deposited in the circumferential direction to reflect the mechanical properties of the leaflets in the circumferential direction. Therefore, depositing the first and/or second set of fibres 12/18 in specific orientations can be used to form soft tissue scaffolds that act as structural analogues for native collagen extra cellular matrix supports.

Because the features of the sheet 10 are relatively fine for a melt electrowritten soft tissue scaffold, a working distance between the nozzle and the stage usually is less than about 10 mm, but generally the resolution and details that can be deposited are best if the working distance is less than 4 mm.

Although the embodiments and examples have been directed to a soft tissue scaffold for heart valve leaflets, this disclosure extends generally to anisotropic soft tissue scaffolds for use in regenerating tissue such as blood vessels, epidermis, tendon, ligament, breast and other tissue that requires the use of an anisotropic collagen extra cellular matrix, and it is not limited to scaffolds for heart valve leaflets.

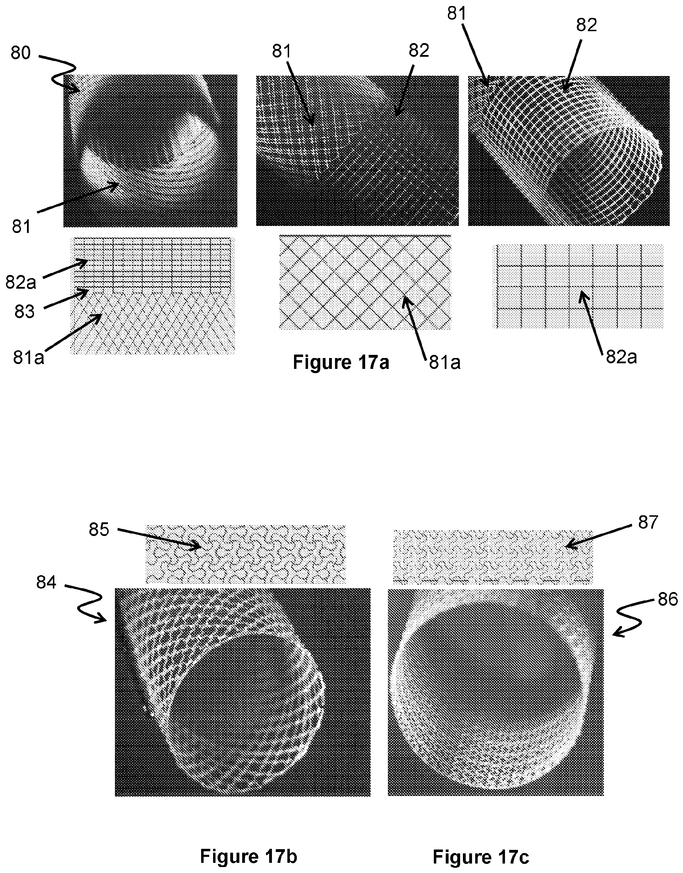

Another embodiment of a scaffold 80 is shown in Figure 17a. Scaffold 80 is a tubular scaffold that has two different regions, in the form of two different scaffold architectures. One region of the scaffold 80 has a first architecture 81 and another region of the scaffold has a second architecture 82. The first architecture 81 has a diamond-type pattern, as represented schematically as 81a. The second architecture has a square mesh-type pattern, as represented schematically as 82a. An interface region 83 is formed at the boundary where the first architecture 81 converts to the second architecture 82. The first and second architecture 81 and 82 are integral in Figure 17a. However, in some embodiments the first and second architectures 81 and 82 are not integral.

Another embodiment of a scaffold 84 is shown in Figure 17b. Scaffold 84 is tubular and has an architecture that is formed from a mesh having serpentine fibres, as represented schematically at 85. Another embodiment of a scaffold 86 is shown in Figure 17c. Scaffold 86 is tubular and has an architecture that is formed from a mesh having serpentine fibres, as represented schematically at 87. In some embodiments the first or second architectures 81 and 82 are replaced with architectures 85 and/or 87. The scaffolds 80, 84 and 86 in one embodiment are formed from PCL fibres having a diameter ranging from about 10 nm to about 100 µm. A distance between adjacent fibres ranges from about 0.1 mm to about 2.5 mm.

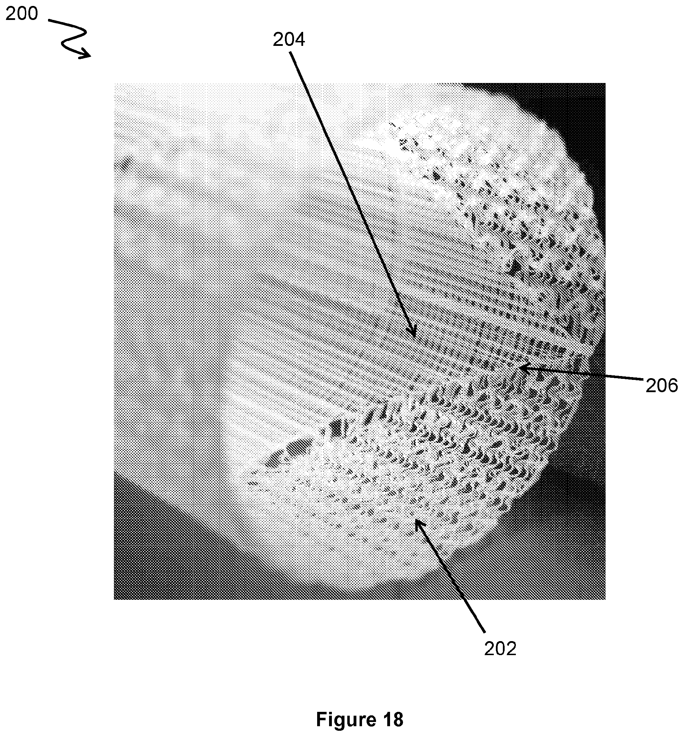

Another embodiment of a scaffold 200 is shown in Figure 18. Scaffold 200 has a first region 202. The first region 202 is formed from a melt electrowritten anisotropic soft tissue scaffold. The anisotropic soft tissue scaffold of the first region in some embodiments is that as described with reference to Figures 1 to 13. For example, the first region can have an architecture of sheet 10. Extending from the first region 202 is second region 204. The second region 204 is formed from a melt electrowritten material. The second region 204 acts as a support for suturing the scaffold 200 to tissue, such as into the aortic root. The second region 202 may be a soft tissue scaffold. The architecture of the second region 204 is chosen to provide good suture retention properties. In some embodiments, the architecture of the second region is selected to provide, in use, pulsatile behaviour that matches the aortic root. In some embodiments the second region 204 is isotropic. For example, the second region 204 can have a polymer architecture similar to 82a. In some embodiments the second region 204 is anisotropic. An isotropy of the second region 204 may be adjusted by adjusting a relative angle between the different set of fibres. For example, an isoptropic material may be formed when the first and second set of fibres are arranged at about 90º relative one another, but an anisotropic material may be formed is the first and second set of fibres are arranged at an angle >90º relative one another. The transition between the first and second regions 202 and 204 is defined by interface 206. When the first and second regions 202 and 204 are integral the interface 206 is formed by a change in fibre orientation.

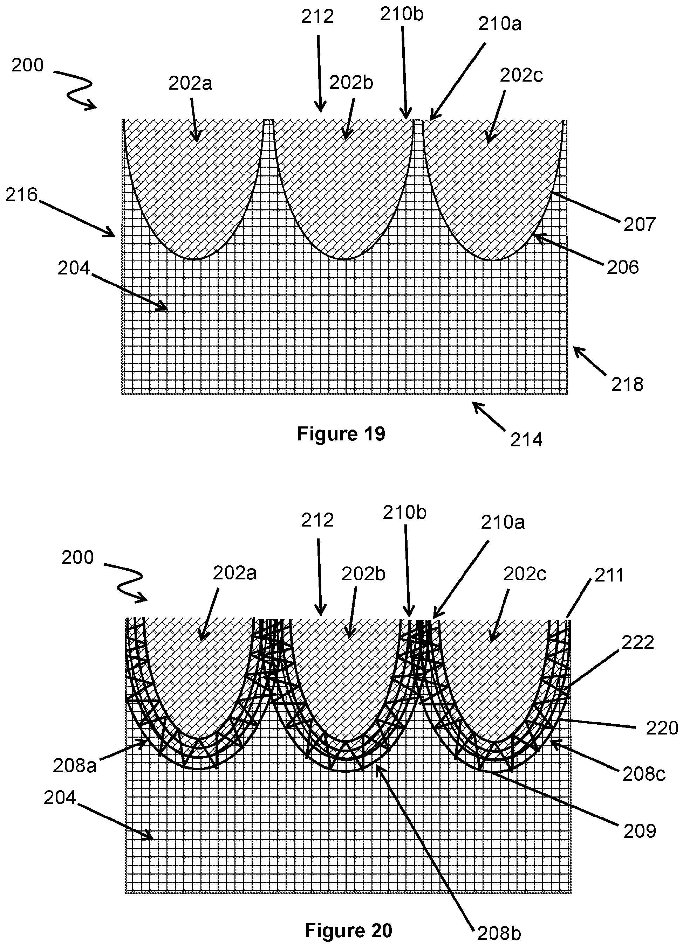

A schematic representation of the tubular scaffold of Figure 18 is shown in Figure 19. The scaffold 200 represented in Figure 19 is a planar projection of the tubular structure shown in Figure 18. In some embodiments the scaffold 200 is prepared as a planar sheet that is then rolled and joined to form a tubular structure. For example, edge 216 and edge 218 can be joined together to form a tubular structure. However, in some embodiments the scaffold 200 is prepared as a tube. In the embodiment of Figure 19, the scaffold 200 has three first regions 202a, 202b and 202c. The first regions 202a-202c are semi-circular in shape. The second region 204 extends from the first regions 202a-202c. The boundary 206 between each of the first regions 202 and the second region 204 is formed at the curved edge 207 of the first region 202.

The term "region" is to be interpreted broadly to mean an area with a similar polymer architecture. For example, the first region has a polymer architecture that is anisotropic, and the second region has an architecture that is isotropic. Generally, the architecture of each of the first regions 202 is the same, but in some embodiments they may differ. For the purpose of explaining embodiments of the disclosure, the first and second regions depicted in Figures 19 and 20 are represented by different cross-hatching, and the architecture of the first and second regions is not limited to the depicted cross-hatching structures.

The first regions 202a-202c form the three heart valve leaflets of the aortic root. The vertices 210 of adjacent first regions, e.g. 202b and 202c, are positioned proximate each other. The vertices 210 are spaced apart from one another so that a portion of the second region 204 is positioned between the vertices of adjacent first regions 202. However, in some embodiments the vertices of the first regions 202 touch and/or overlap with one another. The scaffold 200 has opposing edges 212 and 214. Edge 212 is a downstream edge (e.g. aortic side) associated with the first regions 202a-202c. Edge 214 is an upstream edge (ventricular side) associated with the second region 204.

The scaffold 200 in some embodiments has an intermediate region in the form of reinforcing region 208. The reinforcing region 208 has a series of concentric semicircular fibres 220 that are arranged parallel to one another, and a number of connectors 222 that connect adjacent fibres 220. In use the scaffold 200 is sutured in place to surrounding tissue. The reinforcing region 208 helps to dissipate and withstand forces exerted onto the scaffold 200 at the suturing locations. The reinforcing region 208 also helps to withstand differential forces applied to the first region 202 and second region 204. The reinforcing region 208 is generally positioned at or is superimposed over the boundary 206. The reinforcing region 208 may be integral with the first region 202 and/or second region 204.

The reinforcing region 208 for each of the first regions 202a-b overlaps near edge 210. The intermediate region 208 extends from a vertex of one first region e.g. 202b to the adjacent proximal vertex of the next first region e.g. 202c. Generally, a stiffness of the scaffold will increase at the reinforcing region 208. At the overlap of the reinforcing regions 208, a stiffness of the scaffold may increase past a desirable value. In some embodiments, the reinforcing region 208 is tapered to control a stiffness of the reinforcing region 208. For example, the number of the fibres 220 and/or connectors 222 may be adjusted as the reinforcing region 208 extends from an apex 209 towards the edge 212 at terminus 211. In a tubular form, the terminus 221 positioned between each of the first regions 202 form the corners between adjacent heart valve leaflets. Adjusting the architecture of the reinforcing region 208 can be used to adjust the mechanical properties of the scaffold 200 and the resulting in use characteristics. This can be used to tailor the mechanical properties of the scaffold 200. The scaffold 200 in one embodiment is formed from PCL fibres having a diameter ranging from about 10 nm to about 100 µm. A distance between adjacent fibres ranges from about 0.1 mm to about 2.5 mm.

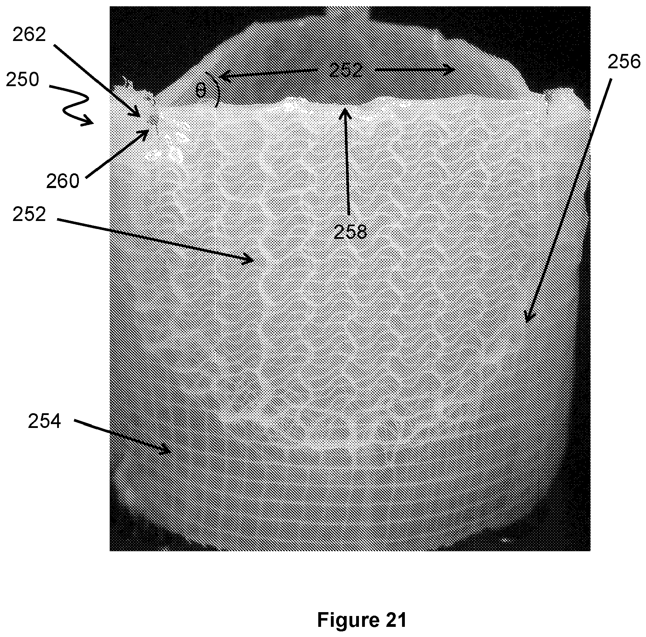

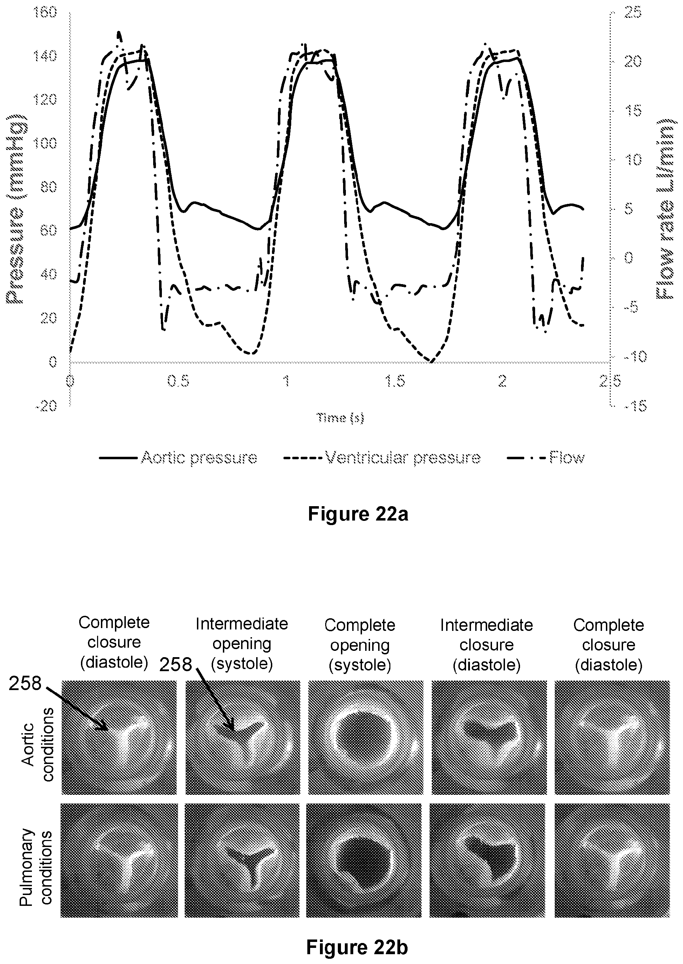

An embodiment of a tubular scaffold 250 having a reinforcing region is shown in Figure 21. The scaffold 250 has a first region in the form of semicircular heart valve leaflet 252 and a second region in the form of tubular body 254. The second region 254 acts as a support to support the first region 252. The scaffold 250 has three heart valve leaflets. The tubular body 254 extends from the heart valve leaflet 252. The reinforcing region 256 is positioned between the heart valve leaflet 252 and the tubular body 254. The heart valve leaflet 252 has a downstream edge (aortic side) 258. In use of scaffold 250, when a backpressure is applied to the scaffold 250, the edges 258 of the three heart valve leaflets 252 come together and engage with one another to close the valve (as best seen in Figure 22b). Sutures 260 connect the vertices of adjacent heart valve leaflets so that an acute angle θ is formed therebetween. The angle θ is similar to that for a native aortic root heart valve. In an embodiment the angle θ ranges from about 30º to about 50º. The angle θ is dependent on the distance from the pinching (suturing) point to the vertex 262 of the first region 252. Forming angle θ helps to ensure the edges 258 come into contact with one another during closure of the valve formed by scaffold 250. In some embodiments the sutures 260 are also used to attach the scaffold 250 to surrounding tissue once the scaffold 250 is implanted. The scaffold 250 in one embodiment is formed from PCL fibres having a diameter ranging from about 10 nm to about 100 µm. A distance between adjacent fibres ranges from about 0.1 mm to about 2.5 mm. In the embodiment of Figure 21, the second region 254 is provided as an isotropic soft tissue scaffold. However, the second region 254 in some embodiments is anisotropic. The second region 254 does not need to be a soft tissue scaffold in all embodiments.

A hydrogel is embedded within the scaffold 250. In some embodiments the hydrogel is an elastin-based hydrogel. The hydrogel may help to promote favourable tissue growth. The hydrogel may also help to withstand mechanical forces applied to the scaffold in use, such as at suturing locations, prior to the formation of tissue *in situ.* In an embodiment, the scaffold 250 is placed into an annulus formed between an inner wall of an outer component and outer wall of an inner component of cylindrical mould, then a hydrogel precursor is injected into the annulus. Once the hydrogel is cured, the hydrogel is embedded in the scaffold. The term "embedded", or variants thereof such as "embed", as used herein it is to be interpreted broadly to mean that tat the hydrogel and scaffold are joined insofar that the hydrogel contacts a surface of the scaffold, and the scaffold can be wholly contained within the hydrogel, the hydrogel can be contained within pores of the scaffold, or a combination thereof.

The hydrogel may either be biologically degradable or biologically non-degradable. Biologically non-degradable hydrogels include polytetrafluorothylene (PTFE) and expanded PTFE, polysiloxanes (silicone, PDMS), thermoplastic polyurethane (TPU), thermoplastic polyurethane urea, polyhedral oligomeric silsesquioxane poly(carbonate-urea) urethane (POSS-PCUU), and/or polysiloxane urethane (urea) (PSU). Biologically non-degradable hydrogels may allow the scaffold to act as a non-degradable replacement heart valve. When the hydrogel is biologically non-degradable, the fibres used to form the scaffold may be biologically non-degradable. When the hydrogel is biologically degradable, the fibres used to form the scaffold may be biologically degradable.

A graph plotting the performance of the scaffold 250 under physiological aortic pressure and flow conditions is shown in Figure 22a. As can be seen, the scaffold 250 shows little backflow and has regurgitation values that agrees with ISO 5840. The relative movement of the heart valve leaflets 252 of scaffold 250 during the simulated physiological aortic pressure and flow conditions is shown in Figure 22b. During maximum flow rate the downstream edges 258 of each leaflet 252 are furthest apart from one another, and during minimum flow rate the downstream edge of each leaflet 252 touch one another to close the valve.

The Figures described specific embodiments in relation to an aortic root valve. However, the polymer architectures and scaffolds of the disclosure can be applied to other valves, such as a vascular valve including a venous valve, and other tissues such as tubular tissue.

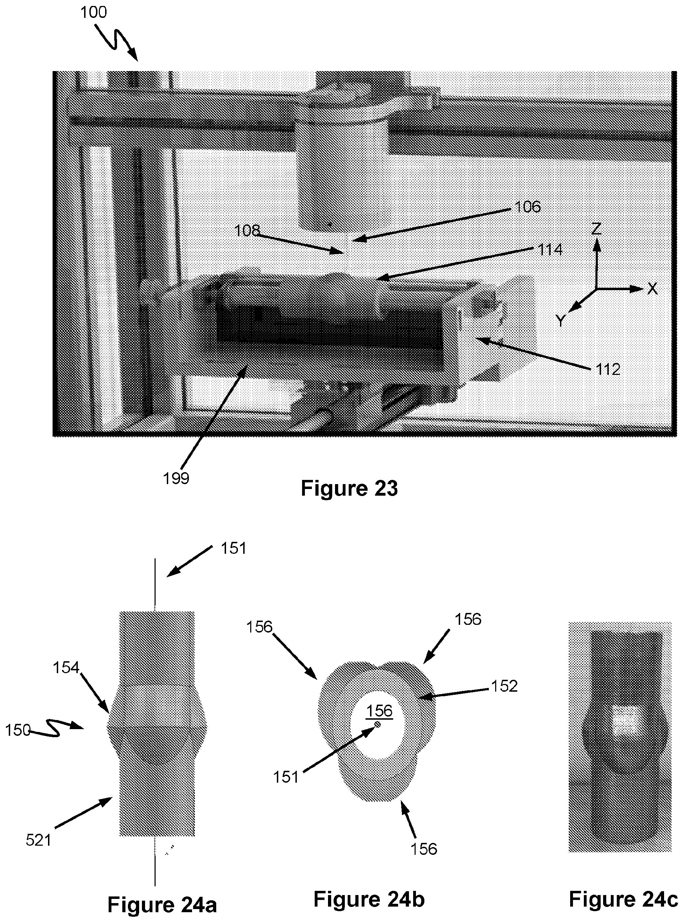

Figure 23 shows an embodiment of a melt electrowriting system 100 for forming a tubular, soft tissue, scaffold. The system uses MEW to form a scaffold structure. The system 100 has a stage 112 to which a conductive mandrel 114 is attachable. Mandrel 114 is conductive to allow a potential to be applied between a nozzle 116 and the mandrel 114. The nozzle 116 allows a polymer to be extruded to form a polymer fibre 118. In the embodiment of Figure 23, the polymer extruded through the nozzle 116 is polycaprolactone (PCL) to form PCL fibre 118. The mandrel 114 is moveable laterally about a plane defined by a base 199 of the stage, i.e. the mandrel 114 can be in an X-Y direction. The nozzle 116 in the system 100 is fixed, therefore the mandrel 114 is moveable relative to the nozzle 116. However, in some embodiments the mandrel 114 is fixed and the nozzle 116 moves relative to the mandrel 114 in an X-Y direction. The nozzle 116 and/or mandrel 114 is also moveable in a Z direction in some embodiments. In some embodiments, the nozzle is rotatable about one or more axis. This means that the nozzle 16 and mandrel 114 are moveable about more than three degrees of freedom in some embodiments.

An embodiment of a mandrel is shown in Figure 24. Mandrel 150 has a longitudinal direction extending along a central longitudinal axis 151 of the mandrel 150. A first segment in the form of tubular section 152 is radially symmetrical. Extending from the tubular section 152 is a second segment, in the form of bulbus region 154. The bulbus region 154 is radially unsymmetrical, which is better viewed from Figure 24b. The bulbus region 154 is formed from three radially extending hemispherical protrusions 156. In the embodiment of Figure 24, the bulbus region 154 is shaped to act as a mould for the three lobes for the sinuses of Valsalva of an aortic root. In the embodiment of Figure 24, a central bore 156 extends along the longitudinal direction 151. The bore 156 allows the mandrel to be coaxially arranged with a shaft associated with the stage 112. It should be appreciated that the mandrel 150 is rotatable, so the associated shaft and mandrel will be in a fixed relationship relative to one another, in use.

The mandrel 150 is conductive. In some embodiments the mandrel 150 is formed from metal. However, in other embodiments, the mandrel is formed of a non-conductive material and rendered conductive by applying a conductive coating to an outside, fibre receiving, surface of the mandrel 150. For example, a mandrel can be prepared using a conventional 3D printer, then a layer of a conductive material, such as copper, be applied to the mandrel, as seen in Figure 24c. When a metal is applied to the mandrel to make it conductive, vapour deposition, sputter coating, etc. can be used. In other embodiments, the mandrel 150 is formed from a 3D printer using a conductive plastic, such as conductive poly(lactic acid)/graphene composite.

The dimensions of the protrusions 156 and their relative size compared to the tubular region 152 is dependent on the size of the scaffold to be formed. For example, a 3D model of an aortic root of a patient can be prepared with sinuses of Valsalva (i.e. the protrusions 156) in accordance to the dimensions described by Thubrikar (European Journal of Cardio-Thoracic Surgery, 28(6), 850-855)*.* This 3D model is then printed using a 3D printer and the resulting structure is made conductive if it is not formed from a conductive plastic. Use of a 3D printer to prepare the mandrel 150 gives rise to patient-specific mandrels so that the resulting scaffold is also patient specific. Other methods of forming the mandrel 114, such as additive manufacturing methods, CNC and casting, can be used to form the mandrel 114.

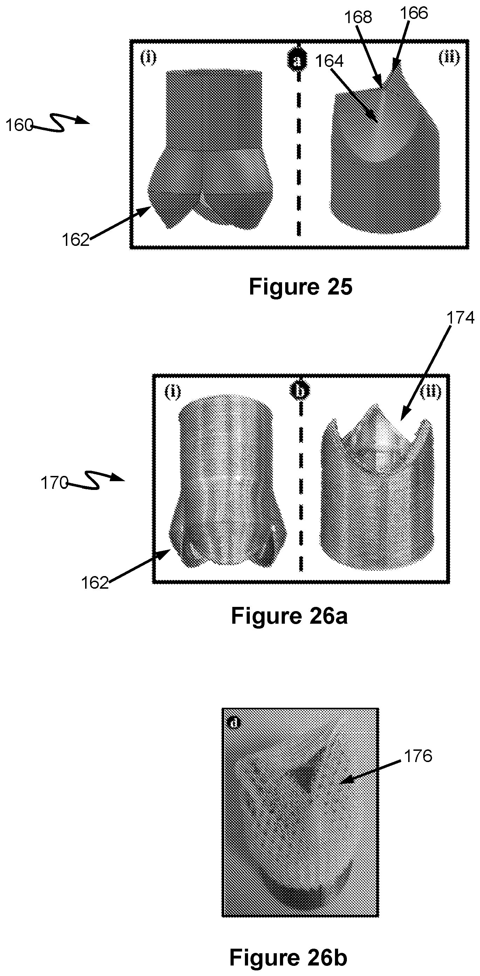

In the embodiment of Figure 24 the mandrel 150 is of unity construction. However, in some embodiment it can be beneficial for the mandrel to be made from two or more segments. This may help to assist with removal of the scaffold from the mandrel once the scaffold has been formed, and it may also help to allow features to be printed in cavities that are formed by the walls of the scaffold. Figure 25 shows a two-part mandrel 160 having a first segment (i) and a second segment (ii). The first segment (i) has a first formation in the form of radially extending flaps 162. The second segment (ii) has a second formation in the form of an inwardly extending concave indents 164. The indents 164 terminate at a ridge 166. Ridge 166 is not continuous so that a gap exists between each ridge line. The ridges generally extend from a common point 168 located near the longitudinal axis. In use, the second segment (ii) is attached to the stage 112 and a fibre 118 is laid onto the mandrel to form a scaffold. The indents 164 form a mould for the valves associated with that sinuses of Valsalva of an aortic root. Once the fibre(s) have been deposited on the indents 164 to form the scaffold for the valves, the first segment (i) is then connected to the second segment (ii) so that the flaps 162 sleeve the indents to be coaxially arranged thereto (not shown). The walls of the aortic root scaffold can then be formed by deposition of the fibre 118 onto the first (i) and second (ii) segment. The first (i) and second segment (ii) are engageable with one another so that they remain in a fixed relationship to one another. For example, an interference fit and/or a bolt can be used to engage the first (i) and second (ii) segments together.

The mandrel 160 is designed using a 3D model of the aortic valve leaflets and root including the sinuses of Valsalva according to the personalized anatomic features of a patient. This model is then collapsed into a two-piece model including the sinuses of Valsalva and the aorta on the outflow side as the first component, and the concave shape of leaflets (indents 164) and aortic wall on the inflow side (left ventricle) as second component. Fibre deposition during tubular MEW formation of the scaffold would facilitate the attachment of tubular scaffold to the leaflet scaffold by fusing on the commissures, inter-leaflet triangle and annulus mimicking the native aortic valve.

An advantage of the mandrel 160 is that the valve and walls of the aortic root scaffold can be prepared using a single mandrel. Further, since the mandrel 160 can be printed using a 3D printer, the geometries of the flaps 162 (which act as a mould for the sinuses of Valsalva) and the indents 164 (which act as a mould for the valves) can be specifically controlled for a patient. This allows the manufacture of custom soft tissue scaffolds. Further, the use of melt electrowriting to form the scaffold means simple and fast manufacturing techniques can be employed.

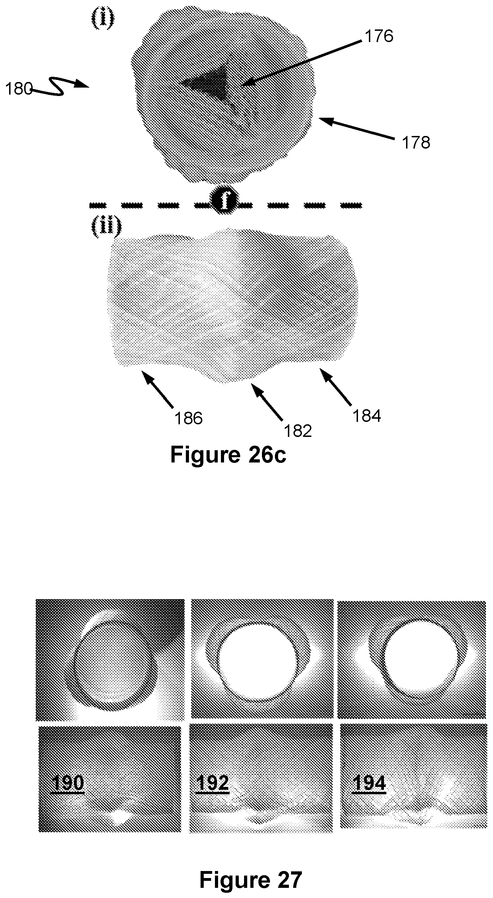

Another embodiment of a two-part mandrel is shown in Figure 26a. In this embodiment, mandrel 170 has a first segment (i) and a second segment (ii) similar to mandrel 160. The first segment (i) has a first formation in the form of radially extending flaps 172. The second segment (ii) has a second formation in the form of semi-circular cutaways 174. To form an aortic base scaffold using mandrel 170, a first component in the form of scaffold mesh 176 is wrapped around the cutaways 174, as best seen in Figure 26b. The first segment (i) of the mandrel 170 is then attached to the second segment (ii) of the mandrel whilst the mesh 176 is held in place. The assembled mandrel is then placed into the stage 112 and the wall and sinus of the aortic scaffold is then formed by deposition fibre 118 onto the mandrel 170. A portion of the mesh 176 becomes incorporated into the wall, fixing the mesh in place relative the wall. An embodiment of a scaffold formed using the mandrel 170 is shown in Figure 26. Figure 26c(i) is a view looking along the longitudinal axis (e.g. 151) showing an embodiment of a scaffold 180 where the mesh 176 is in a cavity formed by the wall 178 around the sinuses of Valsalva 182 (Figure 26c(ii)). Use of a two-part mandrel can assist in removal of the scaffold from the mandrel once the scaffold has been made.

In some embodiments, a coil heater is located in the bore 156 to heat the mesh 176 (i.e. leaflets) close to its melting point while melt electrowriting the wall (i.e. root scaffold) over the top of the mesh 176 to provide a more secure connection between the mesh 76 and wall 180. In other embodiments, a hydrogel system is incorporated on the commissures to help in better attachment of the basal part of leaflets to the wall. This can be done in a post processing step. In other embodiments, local heating of the attachment points facilitates better fusion between the mesh 176 and wall 180. This can be performed by utilizing a small intensity laser to precisely localize the fusion points to the desired locations. It should be understood that more than one form of providing a more secure connection between the mesh 76 (i.e. valve leaflets) and the wall 180 (i.e. root scaffold) can be used in some embodiments.

To form a scaffold using the system 100, the fibre 118 is drawn from the nozzle 116 and deposited (e.g. printed) onto the mandrel 114. At the same time, the mandrel 114 is rotated and moved in the X direction (i.e. along the longitudinal axis of the mandrel) so that the fibre 118 is deposited in a winding manner onto the mandrel 114 at an angle relative to the longitudinal direction 151. In some embodiments, a distance between the nozzle 116 and the outer surface of the mandrel 114 is adjusted by moving the stage 112 and/or the nozzle in a Z direction. The speed at which the mandrel 114 is moved in the X direction determines the winding angle. As the speed in which the mandrel is moved in the X direction increases, the winding angle of the fibre 118 decreases. Conversely, if the speed at which the mandrel is moved in the X direction decreases, the winding angle of the fibre 118 increases. In some embodiments, the speed at which the mandrel 114 is rotated is also changed to adjust the winding angle. Increasing the rotation speed of the mandrel 114 increases the winding angle when a given movement on the mandrel 114 in direction X is kept constant, and decreasing the rotation speed of the mandrel 114 decreases the winding angle. In some embodiments the speed at which the mandrel 114 is moved in the X direction and the speed at which the mandrel 114 rotates is adjusted to control the winding angle. In some embodiments, the mandrel 114 is also moved in the Y direction (i.e. transversely to the longitudinal direction of the mandrel 114) in addition to the X direction. Movement of the mandrel 114 in the X-Y direction can be used to deposit (i.e. print) specific fibre architectures. Additionally, the mandrel can be moved according to predefined coordinates to control the position at which the fibres are deposited (e.g. printed). In other words, fibres may be printed onto the 3D conductive mandrel with specific fibre architectures, such as serpentine arrangements and organic micro architectures. The mandrel 114 is rotated and moved back and forth along the X direction until a wall of the scaffold is formed. A single fibre can be used to form the wall of the scaffold, in which case the wall and any associated features of the wall are unitary with one another. Alternatively, two or more fibres can be used to form the wall. For example, some embodiments use two or more nozzles that form two or more different fibres.

Changing the winding angle helps to control the mechanical properties of the scaffold. The wall 178 around the sinuses of Valsalva 182 is generally formed of fibres deposited at a winding angle of greater than 45°, such as 60°, to help the scaffold 180 withstand radially and circumferentially extending mechanical forces in use of the scaffold 180. A base 184 (i.e. an inflow side of the valve) and top 186 (i.e. an outflow side of the valve) of the aortic root scaffold 180 is formed by winding fibres onto the tubular section 152. The fibre angle of the base is generally less than 45°, such as 30°, to help the scaffold withstand forces acting along the longitudinal axis of the scaffold. Increasing a fibre density of the scaffold also helps to increase the mechanical strength of the scaffold. Generally, the sinuses 182 of the scaffolds are expected to be stiffer compared to the wall (184/186). For example, fibres can be deposited with a smaller fibre spacing on the sinuses of Valsalva and a larger fibre spacing on the aortic wall.

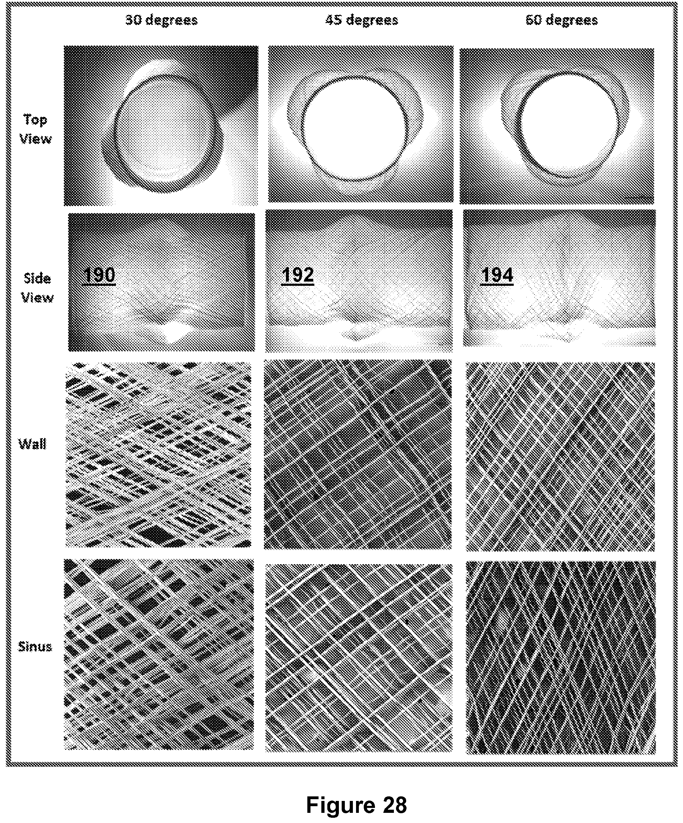

The specific winding angle, a transition between different winding angles, and a length of an area with a specific winding angle will be determined by size of the scaffold 180 and the structural requirements of the scaffold 180. For example, a scaffold for implantation into an adult patient will have different requirements for a scaffold for implantation into a child patient. An example of scaffolds with different dimensions and fibre angles is shown in Figure 27. Scaffold 190 has a fibre at an angle of approximately 30° to the longitudinal direction, scaffold 192 has a fibre angle of approximately 45°, and scaffold 194 has a fibre angle of approximately 60°. Microscopic images of tubular scaffold embodiments of scaffolds with fibres at 30° (scaffold 90) and 45° (scaffold 192) are more clearly seen in Figure 28. The parameters used in system 100 for producing one of the scaffolds shown in Figure 27 is given in Table 1. As the rotational speed of the mandrel is increased, the winding angle increases. In some embodiments a base layer is first deposited (e.g. printed) onto the mandrel 114. A subsequent layer of fibre 118 is then applied directly over the base layer. To help bond the layers together, a temperature that the second or more layers is deposited onto the mandrel 114 is higher than a temperature at which the base layer is deposited onto the mandrel 114. For example, the first layer can be deposited at 81 °C and the second layer can be deposited at 91 °C. Using a higher temperature for the second or more layers helps to fuse the layers together. In some embodiments, the scaffold is annealed to help fuse the various layers together. The embodiments described herein are based on PCL-based fibres. However, PCL is only one example of a polymer that can be used in melt electrowriting to form a soft tissue scaffold as described herein.

**Table 1. System parameters used to produce a scaffold.**

| Winding angle | Speed (rev/min) | Voltage (kV) | First layer temp (°C) | Second layer temp (°C) |
|---|---|---|---|---|
| 30° | 6 | 10.8 | 81 | 91 |
| 45° | 11 | 11.0 | 81 | 91 |
| 60° | 19 | 11.2 | 81 | 91 |

The fibre diameter can also be adjusted by changing the rotation speed of the mandrel 114 and the winding angle. Generally, as the winding angle increases, a diameter of the fibre 118 decreases. In some embodiments, the fibre 118 has a diameter ranging from about 10 nm to about 100 µm. One or more fibre diameters can be used to form a scaffold. The specific fibre diameter(s) can depend on the types of cells to be seeded onto the scaffold and the tissue to be regenerated, and the mechanical property requirements of the scaffold.

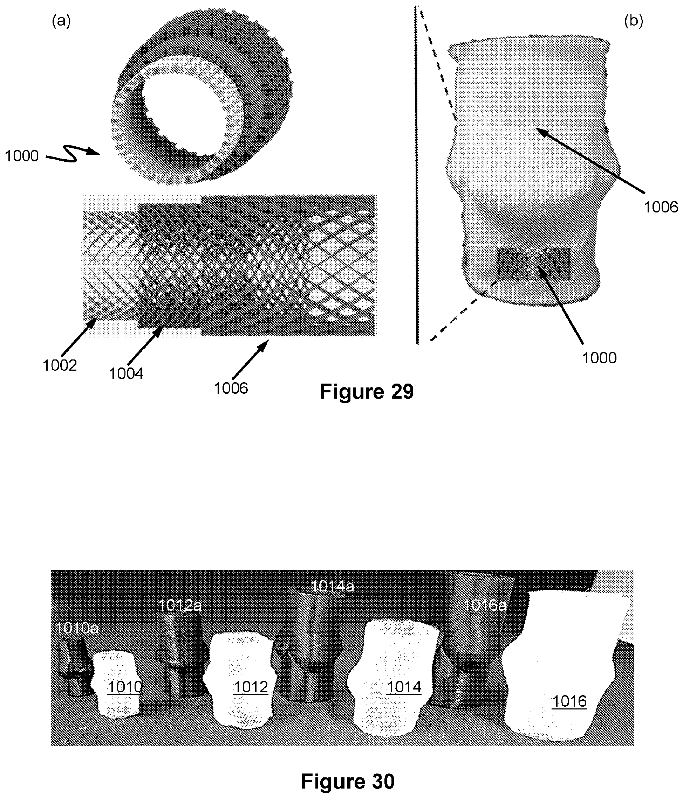

It should be appreciated that a layer of the scaffold can be formed by depositing more than one layer of fibres into the mandrel to form a structure. However, the scaffold can have more than one structure. For example, in some embodiments, more than one structure is deposited onto the mandrel 114. Fibres of each structure can be arranged at a single angle, or at a plurality of angles. An embodiment of a three-layer structure scaffold is shown in Figure 29. Figure 29(a) shows a simulated model 1000 having an inner structure 1002, an intermediate structure 104 and an outer structure 106, each of which being coaxially arranged with one another. The inner structure 1002 has fibres arranged at 50°, the intermediate structure has fibres arranged at 65°, and the outer structure has fibres arranged at 40°. This arrangement is similar to the collagen fibre orientation of a native aorta, where the inner structure 1002 act as a scaffold for the intima, the intermediate structure 1004 acts as a scaffold for the media, and the outer structure 1006 acts as a scaffold for the adventitia. The scaffold 1000 in some embodiments has mechanical properties that resemble a native tissue that the scaffold intends to regenerate. This means that during the initial stages of implantation when the regenerating tissue is still too immature to fully support itself, the mechanical forces experience *in situ* can be transferred to the scaffold. As the cells proliferate and new tissue begins to grow, the scaffold can degrade away when a biodegradable fibre is used to form the scaffold to be replaced by regenerated tissue. During this transition from a scaffold-support tissue to regenerated tissue, the mechanical forces exerted onto the valve *in situ* are progressively transferred from the scaffold to the regenerated tissue.

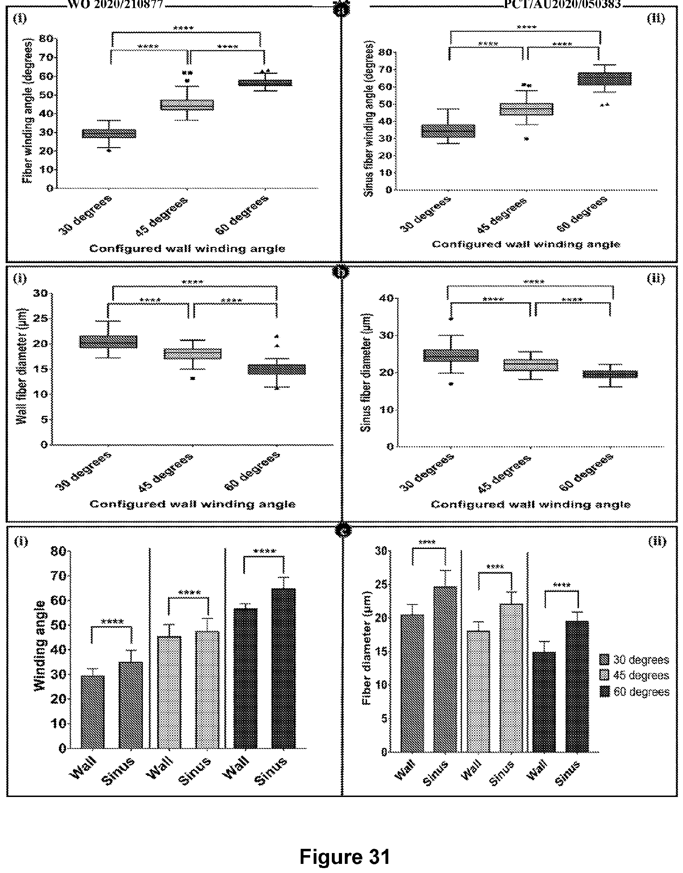

An embodiment of a scaffold having this three-layer structure is shown in Figure 29(b), showing the simulated model superimposed with the outer structure 1006. The size and features of the scaffold 1000 is dependent upon the size and feature of the mandrel onto which the fibres are deposited, and the requirements for the specific patient. Figure 31 shows various three-layered structured melt electro-spun scaffolds. Scaffold 1010 has an aortic wall with a diameter of about 10 mm, scaffold 1012 has an aortic wall with a diameter of about 15 mm, scaffold 1014 has an aortic wall with a diameter of about 20 mm, and scaffold 1016 has an aortic wall with a diameter of about 25 mm. The respective mandrel (110a, 112a, 114a and 116a) are also shown.

Although the embodiments and examples have been directed to aortic root scaffolds, this disclosure extends generally to tubular soft tissue scaffolds such as blood vessels and is not limited to aortic root scaffolds.

### Examples

Exemplary embodiments will be described by way of example only.

### Example 1

### 1.1Material and methods

### 1.1.1 Material selection and scaffold design rational

PCL is chosen as the candidate for this application due to its slow degradation profile which provides the required time for the secretion of ECM proteins and tissue development prior to the degradation of scaffold and loss of mechanical integrity. Biocompatibility and relatively inexpensive production route of this polymer provides a promising foundation for HVTE applications. In addition to the material properties fibre alignment, porosity, fibre diameter and hierarchical microstructure are contributing factors to the anisotropic mechanical properties as well as biological activities of the scaffold including cell attachment, infiltration, and differentiation and ECM production. These factors have to be carefully considered in the design and fabrication of a scaffold for heart valve tissue engineering. Leveraging the capabilities of Melt Electrowriting (MEW), scaffolds with controlled and predefined structure, porosity and fibre diameter can be designed and fabricated for the aortic heart valve position. For this purpose, biologically inspired electro-spun fibres are designed to mimic the wavy-like orientation of collagen fibres apparent in the Fibrosa and Ventricularis layer recapitulating the composition, dimensions and mechanical properties of the native aortic valve leaflet while providing a biomimetic structure for extracellular matrix (ECM) deposition.

### 1.1.2 Fabrication of biomimetic scaffolds

Biologically inspired scaffolds are fabricated with an in-house built Melt Electrowriting (MEW) and schematically illustrated in Figure 23. MEW is an emerging scaffold manufacturing technique which enables the fabrication of solvent-free scaffolds by combining electrospinning and additive manufacturing principles. In this process, medical grade PCL pellets (Purasorb^{®} PC 12, Purac Biomaterials, The Netherlands) are heated at 75 and 85 °C in a plastic syringe (Source). 2.0 bar of air pressure pushes the molten polymer through a 23 G needle where high voltage of 6-6.5 kV drags the fibre down onto a laterally translating aluminum collector. The needle was initially kept at 7 mm from the collector and reduced to 4mm for the samples that were tested under dynamic conditions as better accuracy of deposition could be achieved by melt electrowriting at a lower gap. When the needle was kept at 4 mm from the collector, the stage was moved at 280 mm/min as better accuracy of deposition could be achieved by electrowritting at a smaller gap and slower collection speed compared to studies performed previously. All fibrous networks (80mm × 20mm × 0.5mm) are cut into (20mm × 10mm) samples with a laser cutting machine (ILS12.75, Universal Laser Systems, Inc. USA) at 80 W to be used for mechanical characterization, imaging and cell seeding.

### 1.1.3 Morphological characterization with imaging techniques

The morphological properties of scaffolds were analysed by Scanning Electron Microscopy (SEM, JSM, 7001f, JEOL Ltd, Japan). PCL melt electro-spun samples were gold sputter coated (JEOL fine sputter coater) for 150s at 10mA prior to imaging and observation was made at 32mm of working distance, 10 kV and under vacuum conditions. The global view, fibre stacking and fusion points are looked at in the imaging process as these are the determinant factors for the quality of the print. A stereomicroscope (Leica M125, Leica Microsystems, Germany) was used to evaluate the fibre diameter and alignment of fibres through the process of printing optimization (n=20).

### 1.1.3 Characterization of mechanical properties

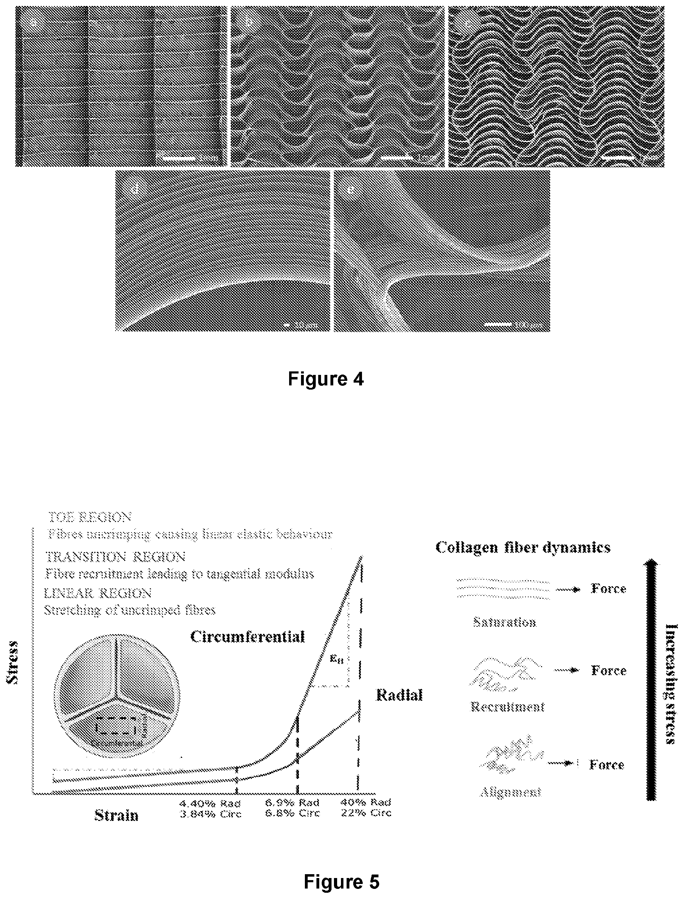

Uniaxial tensile testing was performed on all groups of scaffolds using an Instron Micro Tester equipped with a 500N load cell (5848, Instron, Australia). Samples (n=5) were secured with pneumatic pressurized clamps in circumferential direction and suspended in air at room temperature. A tensile strain of 100% of the scaffold's height was applied at a strain rate of 0.1 mm/s and a stress/strain curve was plotted to characterize the effect of pore-seize, layer number and degree of curvature. Linear elastic modulus, tangent modulus and high tensile modulus of all samples was calculated from the slope of stress/strain curves at initial linear region (0-5%), transition region (15-20%) and steepest region of curve (20-30%) respectively. The maximum stress at the peak point was noted and represented as Ultimate Tensile Stress (UTS) and was compared with maximum stress at failure of the native aortic valve leaflet. The scaffold that best represent the mechanical properties of the native aortic valve leaflet was then chosen for further mechanical testing. Samples were laser cut in the radial direction (illustrated in representative Figure 5) at (20mm × 10mm) to measure the anisotropic ratio of the scaffold. The group that best mimicked the anisotropy of native leaflet tissue was plotted in a stress/strain curve and was selected for a thorough dynamic mechanical testing including step-wise stress relaxation, fatigue and hysteresis tests performed with samples submerged in phosphate buffered saline (PBS) at physiological conditions (37 °C).

Step-wise stress relaxation test was performed to evaluate the behaviour of the selected PCL melt electro spun scaffold under equilibrium conditions. The samples were subjected to 10 % of ramp tensile stretching steps at 0.1 mm/s strain rate and kept constant for a duration of 15 minutes between each step. The stress relaxation behaviour was observed even beyond 15 minutes of relaxation period, but a threshold of 0.0001N was initially defined to identify the relaxation period for the stress relaxation test. The equilibrium modulus was calculated from the slope of stress/strain curves plotted from the stress relaxation test.

Mechanical fatigue is of high importance in the context of valvular biomechanics due to the repetitive stress applied during systolic and diastolic cardiovascular cycles. Fatigue properties were investigated on a uniaxial tensile testing setup where samples were subjected to a sinusoidal tensile strain at an amplitude of 10% and frequency of 1 hertz for 500 repetitive cycles. The frequency and amplitude used for this fatigue test fully replicate the cardiovascular loading conditions as the tensile forces are applied at 70 beats/min (equivalent to 1Hz) at which it stretches an aortic valve leaflet up to 10% of its initial length. The scaffold stiffness at the first cycle and every 100 cycles was reported to measure the stiffness deterioration of scaffold under fatigue conditions. Moreover, the scaffold stiffness was reported with respect to the number of force cycles applied on the scaffold in order to characterise the trend at which this electro-spun scaffold degrades.

Other important viscoelastic characteristics hysteresis and recoverability are characterized to be compared with porcine aortic valve leaflet viscoelastic properties published by Anssari-Benam et al.² Hysteresis test is performed by incremental loading and unloading 5% cycles to a maximum of 40% of the initial length. Samples are first loaded to 5% of initial length at 0.1 mm/min strain rate and then brought back to starting point. This is then repeated by stretching the sample up to 10% and continuously repeated to identify the point where large energy dissipation is observed and scaffold fails to fully recover its initial length.

### 1.1.4 In vitro biological characterization

### 1.1.4.1 Cell isolation and culture

Human umbilical cord vein smooth muscle cells (HUVSMCs) were isolated from umbilical cords kindly provided by the Department of Gynecology at the University Hospital Aachen in accordance with the human subjects' approval of the ethics committee (EK 2067). HUVSMCs were isolated by stripping out the umbilical cord, removing the remaining adherent connective tissue, cutting 1-mm tissue rings and placing them in cell culture flasks. Outgrowth of HUVSMCs from the tissue rings onto the tissue culture plastic (TCP) was observed after 1-2 weeks. HUVSMCs were cultured in Dulbecco's modified Eagle medium (DMEM; Gibco) supplemented with 10% fetal calf serum (FCS; Gibco) in 5% CO₂ and 95% humidity at 37°C up to a confluence of 80% to 90% and subsequently passaged. Cells between passages 5-7 were used for seeding the MEW scaffolds. Prior to seeding, cellular phenotype was verified by immunocytochemical staining for alpha-smooth muscle actin (α-SMA) and von Willebrand factor (vWF), whereby the cells had to be positive for α-SMA and negative for vWF. For this reason, cells were seeded in 96-well plates, fixed in methanol-free 3% paraformaldehyde (PFA; Roth) in phosphate buffered saline (PBS; Gibco) for 30 min and rehydrated in PBS. Nonspecific epitopes were blocked and cell membranes were permeabilized using 5% normal goat serum (Dako) in 0.1% Triton-PBS for 1 h at room temperature. HUVSMCs were incubated for 1 h at 37°C with mouse anti-α-SMA (A 2547; Sigma) diluted 1:400, or rabbit polyclonal anti-human vWf (A0082; Dako) diluted 1:200, as primary antibodies. The samples were then washed and incubated with the corresponding secondary antibodies for 1 h at 37°C: Alexa Fluor 594 goat anti mouse (A 11005; Invitrogen), or Alexa Fluor 488 goat anti rabbit (A 11008; Invitrogen), each diluted 1:400. Counterstaining was performed with 4',6-diamidino- 2-phenylindole (DAPI) nuclei acid stain (Molecular Probes). Stained cell-seeded MEW scaffolds were observed with a microscope equipped for epi-illumination (AxioObserver Z1; Carl Zeiss GmbH). Images were acquired using a digital camera (AxioCam MRm; Carl Zeiss GmbH).

### 1.1.4.2 Fibrin synthesis

Lyophilized fibrinogen (Calbiochem) was dissolved in Milli-Q purified water and dialyzed against tris-buffered saline (TBS; pH 7.4) overnight using a 6000-8000 molecular weight cut-off membrane (Novodirect). The resulting fibrinogen solution was filter sterilized, and the concentration was determined by measuring the absorbance at 280 nm using an Infinite M200 spectrophotometer (Tecan Group Ltd). The fibrin gel components of this construct (5.0 mL in total) consisted of 2.5 mL fibrinogen solution (10 mg/mL), and the fibrin polymerization starting solution composed of 1.75 mL TBS containing 5x10⁷ umbilical artery SMC/FB cells or AD-MSCs, 0.375 mL 50 mM CaCl-2 (Sigma) in TBS, and 0.375 mL 40 U/mL thrombin (Sigma).

### 1.1.4.3 Cell seeding experiments

MEW scaffolds were sterilized by dipping in 80% ethanol followed by evaporation inside the biosafety cabinet. After being completely dried, the MEW scaffolds were placed in custom-made silicone (M 4641-A; B&G Faserverbundwerkstoffe GmbH) cell seeding molds. HUVSMCs were enzymatically detached from the TCP by 0.25% trypsin/0.02% EDTA solution (Gibco), collected in a conical tube (Sarstedt) and counted using a Neubauer chamber. Cells were centrifuged at 500 x g for 5 min and resuspended in cell culture medium at a concentration of 12.5 million cells/mL medium. Four spots per scaffold (A = 4 cm²) were seeded, each with 1 million cells in a volume of 80 µL (total of 4 million cells per scaffold).

For the embedding of the MEW scaffolds in fibrin gel, the cells were resuspended in the polymerization starting solution at a concentration of 20 million cells/mL. The mold was filled with the fibrin gel components. The rapid polymerization of the fibrinogen ensured a homogenous cell distribution throughout the graft. The final cell concentration was 10 million cells/mL fibrin gel.

The seeded and fibrin-embedded scaffolds were cultivated for one and two weeks in DMEM supplemented with 10% FCS, 1% antibiotic/antimycotic (ABM; Gibco) and 1 mM L-ascorbic acid 2-phosphate (Sigma) in static conditions at 37°C and 95% humidity. The medium was changed every 2-3 days.

### 1.1.4.4 Live/Dead staining

Cellular viability on the MEW scaffolds after one and two weeks was assessed by a live and dead (LD) staining using calcein AM and propidium iodide. Calcein was used to stain viable HUVSMCs green, whereas propidium iodide was used to label dead cells red. Samples were stained for 10 minutes at 37°C followed by a washing step with PBS. Subsequently, stained samples were observed with a microscope equipped for epi-illumination (AxioObserver Z1; Carl Zeiss GmbH). Images were acquired using a digital camera (AxioCam MRm; Carl Zeiss GmbH).

### 1.1.4.5 Scanning electron microscopy

To investigate cell adherence to and cell coverage and spreading on the MEW scaffold scanning electron microscopy was performed after both culture periods. Cell-seeded MEW scaffolds were fixed in 3% glutaraldehyde in 0.1 M Sorenson's buffer (pH 7.4) at room temperature for 1 h. Afterwards, they were washed with sodium phosphate buffer (0.2 M, pH 7.39, Merck) and dehydrated consecutively in 30%, 50%, 70% and 90% ethanol and then three times in 100% ethanol for 10 min. Samples were critical point dried in CO₂, followed by sputter-coating (Leica EM SC D500) with a 20 nm gold-palladium layer. Images were obtained with an ESEM XL 30 FEG microscope (FEI, Philips, Eindhoven, the Netherlands) with an accelerating voltage of 10 kV.

### 1.1.4.6 Immunohistochemistry

To perform immunohistochemical analysis of the cell-seeded scaffolds, samples were fixed in methanol-free 3% PFA in PBS for 1.5 h at room temperature and washed with PBS afterwards. Fibrin-embedded samples were dehydrated, embedded in paraffin and sectioned. Unspecific epitopes were blocked and cell membranes were permeabilized by 5% normal goat serum (NGS; Dako) in 0.1% Triton-PBS for 1 h at room temperature. Seeded scaffolds were incubated for 1 h at 37°C with the following primary antibodies: mouse anti-human α-SMA (A 2547; Sigma) diluted 1:1000, rabbit anti-human collagen type I (R 1038, Acris) diluted 1:300 and rabbit anti-human collagen type III (R 1040, Acris) diluted 1:50. Samples were washed and incubated for 1 h at room temperature with the following secondary antibodies: samples stained for α-SMA were incubated with a Alexa Fluor 594 goat anti-mouse (A 11005, Invitrogen) antibody and samples stained for collagen type I with a Alexa Fluor 488 goat anti-rabbit (A 11008, Invitrogen) antibody both diluted 1:400 for 1 h at 37°C. Collagen type III stained samples were incubated with a rabbit immunoglobulins/biotinylated (E 0432, Dako) diluted 1:300 for 1 h at 37°C followed by incubation with streptavidin/TRITC (RA 021, Acris) diluted 1:1000 for 1 h at 37°C. The native human umbilical cord served as a positive control. For negative controls, samples were incubated in diluent and the secondary antibody only.

Actin staining was performed according to the manufacturer's instructions. PFA-fixed samples were washed with PBS, cells were permeabilized with 0.1% Triton-PBS for 1 h at room temperature and incubated with a 3.5 nM phalloidin in PBS for 1 h at room temperature. All samples were counterstained, and images were taken as described above.

### 1.1.5 Statistical analysis

Mechanical properties of all constructs are reported as mean± standard deviation. An unpaired T test was used to compare the scaffolds with variable pore-size (n=5), and one-way ANOVA test with a Tukey multiple comparison component was utilized to investigate the effect of layer number and curvature degree (n=3) (GraphPad, Prism 7). Values of p<0.05 were considered significant and the (p<0.001****,0.0001<p<0.001***,0.001<p<0.01**,0.01 <p<0.05*) was used to indicate the level of significance in all bar plots.

### 1.1.6 Valve functionality test setup

A custom-made flow loop system was used to assess the functionality of valves at physiological aortic conditions (flow rate: 5.0 L min-1, frequency: 70 bpm, mean aortic pressure: 100 mmHg, 120-80 mmHg) to assess the mean pressure gradient and effective orifice area (EOA). Pressure transducers (DPT 6000, pvd CODAN Critical Care GmbH) positioned immediately at the inflow and out flow side of the valve were used to measure the pressure and a flowmeter (sonoTT, em-tec GmbH) was utilized to measure the instantaneous inflow to the valve. A LabVIEW application was then used as an interface to record the pressure and flow values measured by the pressure transducer and flowmeter. The ventricular and aortic pressure difference and root mean square of inflow was calculated from ten cycles to identify the mean pressure gradient and EOA according to ISO 5840-2 guidelines.

### 1.2 Results and Discussion

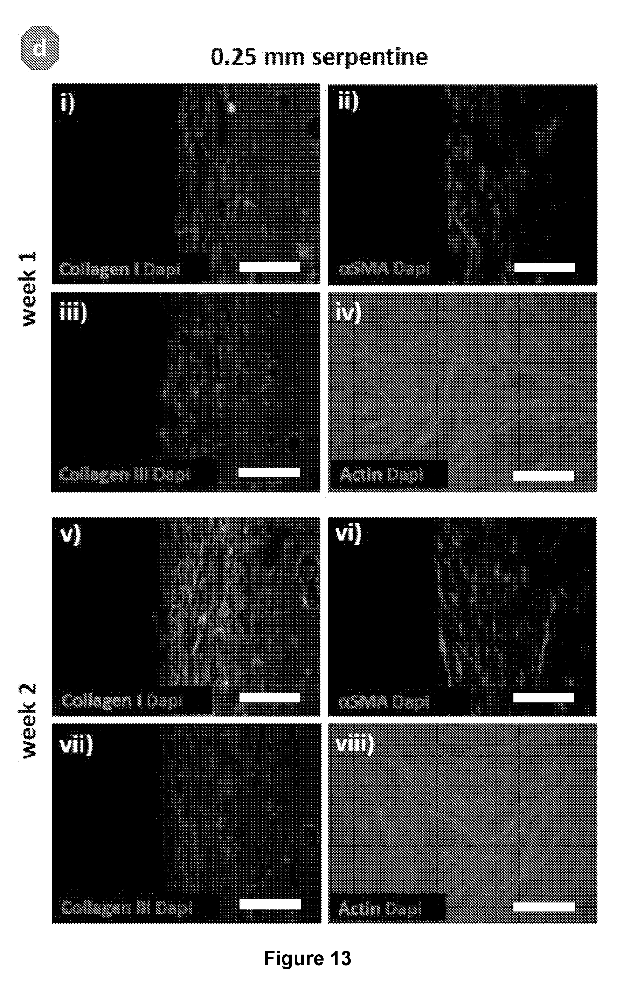

The scaffold architecture mimics the collagen fibres seen in the fibrosa and ventricularis layer of the aortic heart valve leaflet where helical patterns with a 1mm diameter are defined as the lay down pattern for the fibres in circumferential direction (Figure 5, Figure 13). Helically patterned fibres are spaced at 0.5 and 0.25 mm in the circumferential direction to quantify how fibre spacing affects the stiffness of final construct. Collagen fibres are available at a lower density in combination with highly crimped elastin fibres in the radial direction of the native valve leaflets resulting into an anisotropic behaviour. Accordingly, semi circles with 0.5 mm of diameter are designed at a larger spacing (2 and 1 mm) to control and mimic anisotropy. Moreover, 10, 20 and 30 layers of fibres are stacked to characterize the effect of layer number on tensile properties and its correlation with the native leaflet properties. Functional properties of the native aortic valve are associated with the J shaped stress/strain curve and the strain at which maximum stress occurs. To replicate this behaviour and control the strain rate at which the rise in stress occurs, the degree of curvature that the fibres are deposited is controlled and scaffold are fabricated to find the most suitable architecture in accordance with native leaflet properties.

### 1.2.1 Morphology and biological inspired scaffold architecture

The morphology and print quality of straight and helically patterned scaffolds with 0.5 & 0.25 mm circumferential fibre spacing are illustrated with representative SEM images shown in figure 4. Regardless of the scaffold architecture, the average fibre diameter was measured to be 19.76 ± 1.54 *µm* across the constructs. This fibre diameter is a degree of magnitude smaller than scaffolds fabricated with other melt extrusion techniques including Fused Deposition Modelling (FDM) and bio-extrusion that are generally have fibre diameters larger than 200 *µm*. Fibres are accurately stacked across the deposited layers for all groups of scaffolds irrespective to the curve or straight fibre architecture (Figure 4a, Figure 4c). There are small number of fibres found crossed over the intended stacking architecture for the case of 0.25 mm fibre spacing which is due to the electrostatic charges stored during the MEW process. A distinct fusion behaviour is observed for the case of scaffold with helical patterns at 0.5 mm fibre spacing where circumferential and radial fibres are laid on top of each other rather than a single fusion point apparent in scaffolds with straight fibre architecture. Upon proceeding onto dynamic mechanical characterization for 0.25 mm spaced helically patterned scaffolds, larger fibres (28.62 ± 0.87*µm*) were observed because of reducing the needle to collector distance. Better melt electrowriting quality and superior mechanical properties was achieved as result of this change in MEW parameters as it is further discussed in dynamic mechanical characterization sub-section.

### 1.2.2 Mechanical properties of scaffolds with varying pore-size, layer number and degree of curvature

Scaffolds fabricated for heart valve tissue engineering applications are required to withstand mechanical loading conditions applied by cardiovascular flow regimes while allowing for a deformation profile that would give rise to successful opening and closure of valve. Heart valve leaflets exhibit J shaped stress strain curve which is known to be determinant to the optimal function of this soft tissue.

Uniaxial tensile testing results displayed a J-shaped stress/strain curve for all groups of scaffolds as shown in Figure 6. Helically patterned fibres are first transformed from a semi-circle to a straight orientation (Figure 6) allowing for a large extension at a low applied stress. This transformation profile results in a curved transition from an initial linear low stress relationship to a steep stress/strain profile up until reaching a constant ultimate tensile stress. This characteristic is analogous to that of native aortic valve tissue where wavy and interconnected collagen fibres throughout the tissue are first untangled by twisting and bending followed by transforming from helical patterns to straight fibres leading to a tangential rise of stiffness as shows in Figure 6. Therefore, the tensile modulus calculated for a J shaped stress/strain curve should be analysed in respect to a specified strain level identified during its course of displacement.

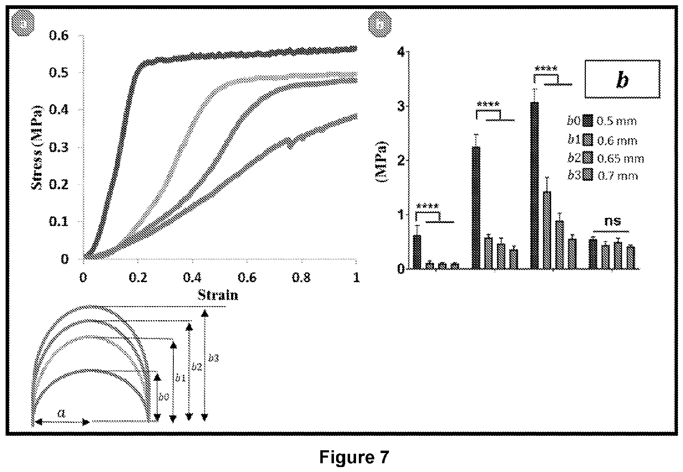

The fibre spacing was found to significantly affect the stiffness at which the UTS was almost doubled from 0.55 ± 0.040 MPa to 0.93MPa ± 0.029 by halving the scaffold pore-size. This substantial increase was also seen in the high tensile modulus value *E*_{*HTM*,0.5 *mm*}= 3.07 ± 0.23 MPa, *E*_{*HTM,*0.25} *ₘₘ=* 4.87 0.094 MPa) whereas the tangential and linear elastic modulus was increased to a lesser degree. This behaviour is explained by the identical curvature patterns used in the fabrication of both scaffolds leading to a similar deformation behaviour but different high and ultimate tensile modulus values (Figure 7 and Figure 6d). On the other hand, the number of stacked layers in the MEW process had a minimal effect on the tensile modulus regardless of the pore-size as the increase in scaffold thickness normalizes the effect of higher forces withstood by the scaffold (Figure 6a). However, the strain at which the maximum stress occurs is dropped by stacking more number of layers since PCL fibres are deposited with a smaller curvature degree in the higher layers as observed in the SEM images shown in figure 4.

To mimic the J shaped stress/strain behaviour of the native aortic leaflet it is crucial to modulate the strain at which the ultimate tensile stress (strain to UTS) is reached. Increasing the curvature degree of designed helical patterns by 0.1mm rises the strain to UTS from an initial 23% to 47% of specimen's initial length. This twofold increase was also observed by increasing the curvature degree with an additional 0.1mm where the scaffold length is double while still retaining a J shaped behaviour. This behaviour is in line with the fact that the scaffold with a higher degree of curvature requires more stretching to straighten the initial curvature like architecture of scaffold in compare with the control group. In addition, a noticeable drop is observed in the tangential modulus for more curved patterns further supporting the change in the curved transition from linear to high tensile modulus caused by the degree of curvature (Figure 7). A slight decrease in UTS is also observed for scaffolds with a higher degree of curvature which is because of drop in the fibre diameter for highly curved fibres though fabricated with similar MEW parameters.

In addition to the J shaped stress/strain behaviour of the aortic valve leaflet, anisotropy is what that allows for more stretchability in the radial direction as opposed to the circumferential direction. Therefore, larger pore-sizes are designed for the radial (1 and 2mm) direction of scaffold to modulate the anisotropic ratio. 1 mm pore-size yields more elasticity where the UTS is 2.56 ± 0.15 times and yield strength is 9.06 ± 0.73 times smaller in radial direction than 0.25 of pore-size in circumferential direction. This ratio rises by two-fold when the radial pore-size is increased to 2 mm. The anisotropic ratio can be modified in accordance with the required level of anisotropy for all of the heart valve leaflets and irrespective of their position.

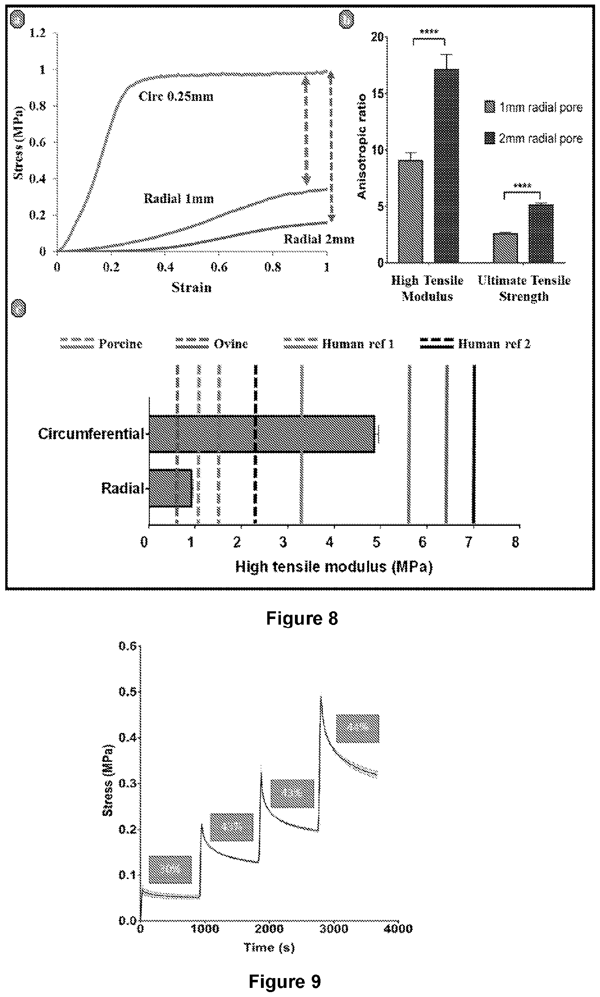

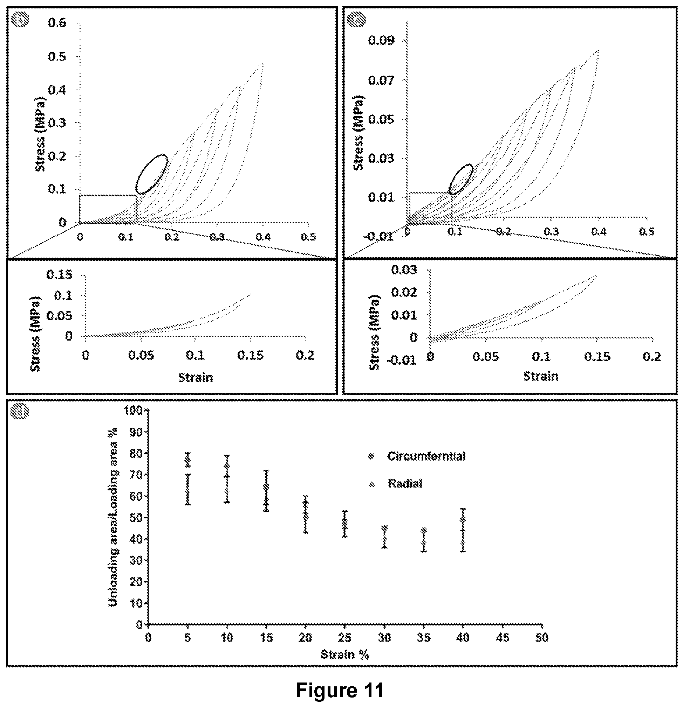

The most suitable architecture, pore size, scaffold thickness, and degree of curvature have to be selected for the scaffold to mimic the mechanical properties of this highly complex tissue. For this purpose, 0.25mm and 2mm fibre spacing was chosen for the circumferential and radial directions, respectively. The J-shaped stress/strain behaviour of this melt electro-spun soft tissue scaffold resembles that of the native valve leaflet of different sources as shown in Figure 11c. Importantly, values of high tensile modulus of a porcine and ovine valve leaflet were fully mimicked with our scaffolds, prior to bioreactor conditioning (Figure 8d). The mechanical properties of the melt electrowritten scaffold came within a close degree of the properties of a human aortic valve leaflet when compared with the results published by Missirlis³. It is still expected that upon cell seeding and dynamic conditioning of this scaffold, a rise in tensile modulus could be observed as a result of the ECM proteins deposition throughout the construct, as it has been reported in other studies.

The most suitable architecture, pore-size, scaffold thickness, degree of curvature and pore-size have to be selected for the scaffold to fully mimic the mechanical properties of this highly complex tissue.

### 1.2.3 Stress relaxation

Stress relaxation has been highlighted as a key characteristic that regulates Cell-ECM interactions for mechanosensitive cell types which should be taken into consideration when fabricating scaffolds as cell culture platforms. Altering the viscoelastic behaviour of biomaterials have been found to effect cell behaviour independent of its stiffness as the cells sense a reduction in the substrate's stiffness. Therefore, a stress relaxation test was performed to assess the viscoelastic behaviour by stretching the scaffold for 10% of tensile strain ramps and allowing the scaffold to relax for 15 minutes at every cycle (Figure 9). The melt electro-spun scaffold exhibited stress relaxation properties where the relaxation behaviour was more prominent at higher strain rates. Rapid relaxation was observed initially and was followed by a slowing relaxation rate at all strain levels. 30% of relaxation was observed after stretching the scaffold for 10% at which it was increased to 44% for increased strain values of stretching. The mechanical response of the melt electro-spun scaffold resembled the relaxation behaviour of a native aortic valve leaflet which has been reported as 27.5 ± 0.77% in a biaxial setup by Stella et al. The stress relaxation values reported for the native leaflet would correlate with stress relaxation behaviour of the melt electro-spun scaffold at 10% strain rate that is how much an aortic leaflet would stretch at physiological blood pressure. This stress relaxation resemblance would provide a native like environment for the seeded cells facilitating mechanical stimulation with the right set of loading conditions.

### 1.2.4 Fatigue

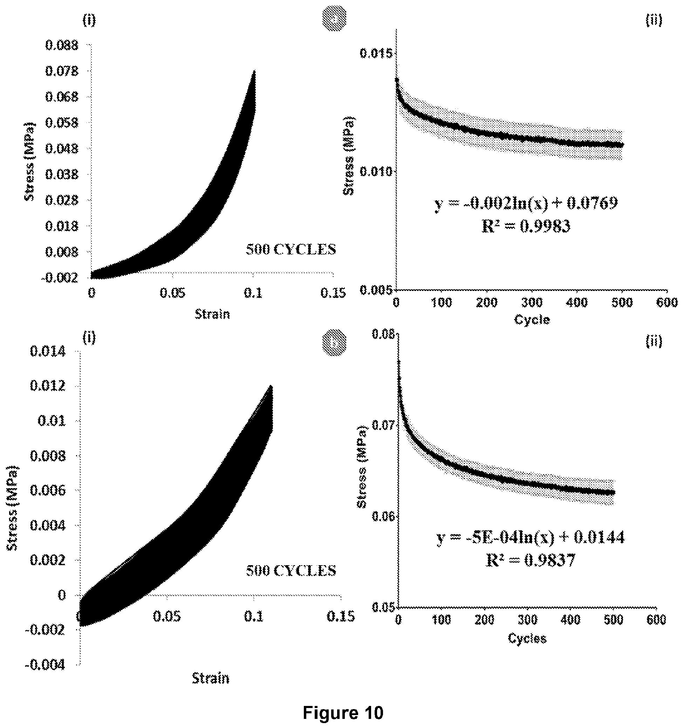

Mechanical fatigue plays a vital role in valvular biomechanics as the valve undergoes a combination of shear, flexure and stretching loading conditions. An aortic heart valve is positioned in a highly demanding physiological condition where repetitive cyclic stress is applied during its function. Despite the importance of fatigue properties, there is very limited amount of information on the fatigue behaviour of a native heart valve as well as the scaffolds fabricated for heart valve tissue engineering purposes. A cyclic uniaxial tensile test was performed on the fabricated MEW scaffolds according to the cardiovascular loading conditions in both the circumferential and radial directions. A J shaped stress/strain behaviour is observed for both test direction similar to that of the native aortic valve leaflet where the circumferential direction is 8 times stiffer than the radial direction further confirming the anisotropy of the melt electro-spun scaffold (Figure 10). The stiffness deteriorates by 19 % in circumferential direction and 20% in the radial direction as the force is repeatedly applied on the scaffold for 500 cycles where the rate of this drop diminishes after around 70 cycles of conditioning. A logarithmic equation was then fitted to this graph to predict the fatigue properties if the scaffold functions for 60 million cycles, equivalent to 2 years of a valve's function. A 47% and 62% drop in stiffness is calculated for the scaffold when tested in the circumferential and radial direction respectively. Most importantly the stiffness of scaffold after 2 years of function 41.1 kPa is still well above the trans-valvular pressure applied on an aortic heart valve leaflet (80 mmHg equivalent to 10.7 kPa).

### 1.2.5 Hysteresis and recoverability

The viscous effect of an aortic valve leaflet and its correlation with resilience remains largely unknown for both the tissue and tissue engineered heart valves despite its importance for functional properties of the valve. To further characterize the viscoelastic properties of the melt electro-spun scaffold, a hysteresis test was performed by loading and unloading the scaffold in both in both circumferential and radial direction to characterize the resilience of this construct by measuring the energy dissipation at different strain levels. The area under a stress/strain hysteresis loading curve up to a given strain level is basically the energy used to stretch the scaffold for a specified range. Similarly, the area under an unloading curve portrays the recovery of that stored energy by bringing the scaffold back to its initial length. It has been previously shown that the difference between these two values would be the dissipation of this straining energy which in high magnitudes could irreversibly stretch the specimen. As illustrated in Figure 11, a J shaped stress/strain curve was observed for both testing directions and all strain levels further highlighting the J shaped behaviour of the fabricated scaffold. However, a linear deformation behaviour was identified starting from the 4^{th} cycle (15%) in circumferential direction and 5^{th} cycle (20%) for the radial direction of test which have previously been shown as an indicator of plastic deformation. The linear stress/strain behaviour was associated with a sudden decrease in the unloading/ loading scaffold area at the same strain level which further highlights the initiation of plastic deformation at these strain levels. A similar phenomenon was reported by Ansar-Benam et al.² for an aortic valve leaflet tissue where the native leaflet was reported to behave similarly both mechanistically and quantitatively.

### 1.2.6 Evaluation of scaffold biological activity

As PCL MEW scaffolds are hydrophobic after manufacture, the scaffold was first plasma-treated with an O₂/Ar₂ plasma to make their surface hydrophilic. Next, the scaffolds' capability to support human umbilical cord vein smooth muscle cells (HUVSMCs) growth, proliferation and extracellular matrix deposition was evaluated. HUVSMCs were chosen as they have been shown to be appropriate for cardiovascular tissue engineering and are clinically relevant for the pediatric population, which could greatly benefit from tissue engineered heart valves by avoiding the repeated surgeries to accommodate somatic growth. HUVSMCs were seeded in two different configurations: i) direct seeding onto the surface of the scaffold and ii) encapsulated in fibrin and composited in a molding process resulting in the complete embedding of the scaffold in a cell-laden fibrin gel. In both cases, the constructs were maintained in static culture for a duration of one and two weeks.

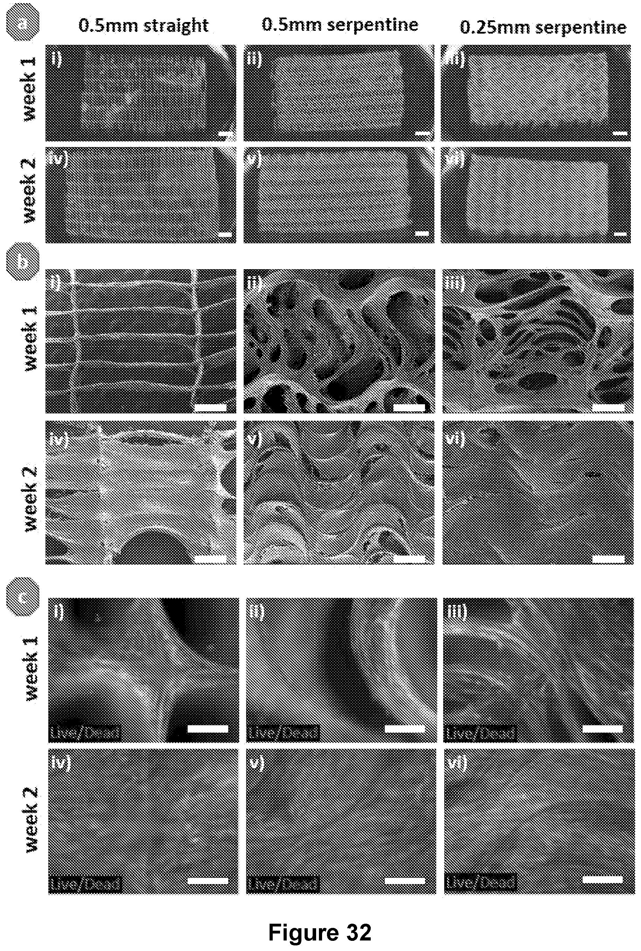

Figure 32 shows the results for the direct seeding approach. The high cell viability shown by the live/dead staining assay (Figure 32c) illustrates the suitability of PCL melt electro-written scaffolds to be colonized by cells. After one week of culture, a small number of pores were bridged by the cells in the serpentine patterned scaffolds but not in the straight fibre scaffolds. This might be attributed to the larger surface area provided by the curvy fibres, which facilitates cell attachment and further colonization. In the following week the pores in all scaffold configurations were confluent (Figure 32b) and the number of dead cells remained low (Figure 32c). Immunohistochemical analysis revealed synthesis of collagen type I and type III, two main components of the native heart valve leaflets.

Next, MEW scaffolds were embedded in HUVSMCs-laden fibrin gels by molding to generate hybrid constructs taking advantage of both components, i.e. tailored mechanical properties and biomimetic microarchitecture provided by the fibre phase, and enhanced extra cellular matrix production typically observed for cell-laden fibrin. The molding process resulted in homogenously embedded MEW scaffolds with no exposed PCL fibres (Figure 12a and Figure 12b) and no negative effect on HUVSMC viability (Figure 12c). Extra cellular matrix deposition of collagen I and III was confirmed by immunohistochemistry (Figure 12d). The remodeling of the hybrid constructs during the 2 weeks of culture did not result in tissue contraction due to the mechanical support provided by the fibres, whereas the control fibrin gels heavily contracted already within the first week (Figure 12aii and Figure 12aiv). Cell-mediated tissue contraction results in leaflets' shortening and, as a consequence, in insufficient valve closure. This well-known phenomenon is a major drawback that jeopardizes the whole concept of engineering a functional tissue both in vitro and in vivo.

### 1.2.7 Valve functionality

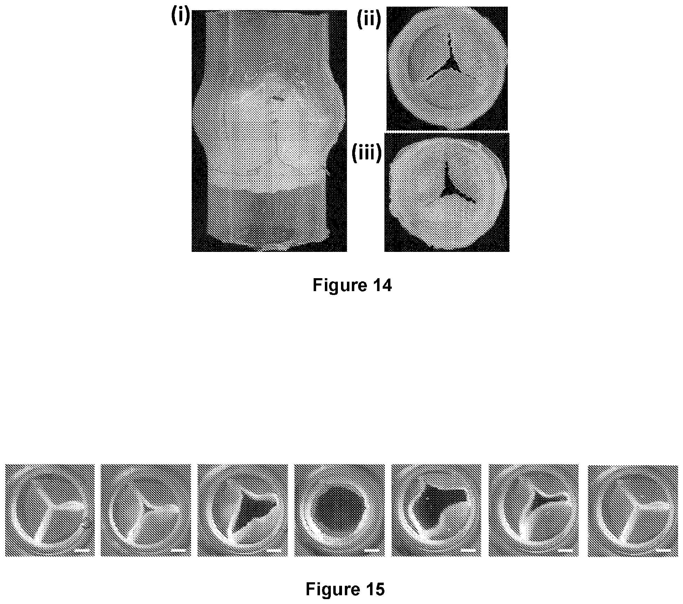

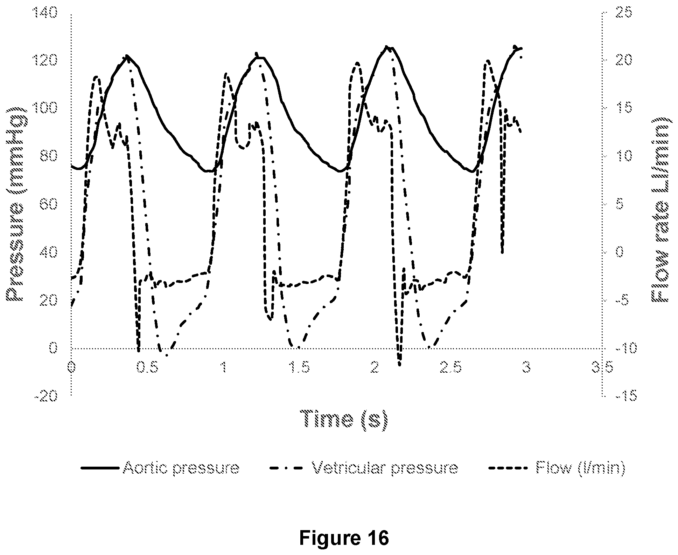

Finally, as a proof of principle, the suitability of MEW scaffolds to be shaped into tri-leaflet valves and their potential to withstand the stringent hemodynamic conditions of the aortic position in a custom-made flow loop system was investigated. The inadequate mechanical properties of tissue engineered heart valves is another major issue which results in most of the valves being implanted in the low-pressure circulation as pulmonary prostheses. MEW scaffolds were embedded in fibrin and sutured as single leaflets into a 2.2 cm diameter silicone model of the aortic root featuring the sinuses of Valsalva (Figure 14) to obtain valves with a 2.2 cm diameter. These valves were tested in a mock-circulation system under physiological aortic pressure and flow conditions as indicated by ISO 5840, and showed a good hydrodynamic performance with a mean transvalvular pressure drop of 2.45±0.36 mmHg and an EOA of 3.3 cm2± 0.26, which meet the ISO requirements for valves with a diameter of 2.2 cm (see Figure 16). This indicated that the scaffolds are strong enough to withstand systemic conditions and at the same time possess adequate bending stiffness to avoid a stenotic behavior. The determination of the bending stiffness has been reported following protocols that were either ad-hoc developed or based on standards for textile characterization, which, however, are affected by technical or conceptual limitations. Therefore, in this study the influence of the layers on the bending behavior of the scaffolds was only shown qualitatively and evaluated the appropriate bending behavior of the valves in the custom-made flow loop. Frames extracted from high-speed movies displayed full closure and unobstructed opening of the leaflets during a simulated cardiac cycle (Figure 15). The hydrodynamic evaluation also demonstrated the suture retention properties and the fact that the valves did not rely on any rigid stent structure to withstand the physiological load. It is important to highlight that the performance of the proof-of-principle valves was achieved with a cell-free fibrin layer and it is expected that dynamic stimulation of a cell laden construct will further improve the mechanical properties of the fibrin. Because of the slow degradation rate of PCL it is expected that in the first *in vivo* phase (18 months), the MEW scaffold will support the *in-situ* tissue formation to guarantee functionality and further development into a mechanically adequate valve upon the following degradation of the scaffold.

### Example 2

### 2.1. Material and methods

### 2.1.1 3D printing of the model for the aortic root

Rapid prototyping using an Fused Deposition Modelling (FDM) 3D printer is chosen for fabricating the mold (mandrel) on which to melt electrospin afterwards, instead of physically manufacturing the mandrel out of a conductive metal to ease and expedite the fabrication process of personalized scaffolds. The aortic root mold was fabricated (Wombat drafter, Australia) by depositing PLA filaments (Bilby 3d, Australia) through a 0.2mm nozzle on a translating collector (1000 mm/min) kept at 90 degrees to help better attachment of model. The resultant model was of high quality with a smooth surface and the dimensions were in harmony with the modeled part. However, conductivity of the collector is a fundamental requirement in the process of melt electrowriting, which is a lacking element in the commonly used materials for FDM 3D printing. Therefore, a conductive layer of copper was deposited on the surface of the model by Physical Vapour Deposition sputtering (PVDS). PVDS coating was performed at a theoretical rate of 8.946 Å per second and 200 Watts by positioning the model in a vacuum chamber (5.1E-7 Torr) for a duration of 2000s where the model was fully coated as a result of this coating protocol (Figure 23c). An alternative to the PVDS coating is to use conductive polymer filaments that can be used to 3D print the desired model. A conductive PLA/Graphene composite (Proto pasta) was used to fabricate the model (Makerbot, Replicator 2x, Australia). The rated volume resistivity of this composite is reported as 15 ohm-cm through the layers. The filament was molten at 210 °C and was deposited on a translating collector moving at 1000 mm/min resulting into a smooth aortic root mould including the sinuses of Valsalva. The latter approach was found to be more suitable to this application as it eliminates the need of post processing coating and expedites the process even a step further. The model is used as a mandrel for MEW to form a 3D scaffold.

### 2.1.2 MEW of personalized tubular scaffolds replicating the macroscopic geometry of aortic root

A custom-made MEW tubular collector was used to fabricate melt electrowritten scaffolds replicating the macroscopic geometry of aortic root including the sinuses of Valsalva. In this process, medical grade PCL pellets (Purasorb^{®} PC 12, Purac Biomaterials, The Netherlands) are heated at 80 °C or 92 °C in a plastic syringe. 2.0 bar of air pressure pushes the molten polymer through a 23 G needle where high voltage of 10.5-11.0 kV drags the fibre down onto a rotating mandrel collector while laterally translating the mandrel in the x axis. The needle was kept at 10.5 mm from the walls of the mandrel, positioning it 7.5 mm from the highest point of sinuses while other MEW parameters are kept constant. Different combinations of rotational and translational speed can be utilized to attain a desired winding angle for the case of a symmetrical aluminum tube¹. However, there are no studies in the literature about MEW on an asymmetrical model made out of a polymer. Moreover, MEW was done on a new mandrel collector assembly where established parameters did not conform to this construct. Although a similar principle was used to establish a relationship between a combination of rotational & translational speed with winding angle for this new collector, MEW parameters had to be optimized to comply with the new collector setup, geometry and conductivity values of the polymer.

To begin with, the effective rotational speed of the motor was experimentally measured as the programmed rotational speed of the motor is not equal to the effective rotational speed of the mandrel collector due to the losses caused by the pulley system. As expected, a linear relationship is observed between the set spindle speed and mandrel rotational speed. This ratio is used to calculate the tangential speed associated with the diameter of the 3D-printed models across the walls and sinuses of Valsalva. The winding angle of fibres is controlled by keeping a constant translational speed (1000 mm/min) while altering the rotational speed of the mandrel. Another important factor to be taken into consideration is the lagging effect of polymer jet on the actual length of deposition as oppose to the programmed tube length. This ratio is used to identify the effective collector translational that directly affects the actual fibre winding angle as previously established in our group. The winding angle of fibres is controlled by keeping a constant effective translational speed (1000 mm/min) while altering the rotational speed of the mandrel (table 1). Fibres on the aortic root are programmed to be aligned at 30°, 45° and 60° with respect to the axis of mandrel. A higher winding angle is expected to be achieved on the sinuses of Valsalva due to the increase in the tangential speed at this area. The voltage applied between the needle and rotating mandrel was slightly increased (by 0.2 kV) for the 45° and 60° scaffolds to account for the additional pull forces applied by the increase in the mandrel rotational speed.

### 2.1.3 Morphological characterization

The morphological properties of the tubular MEW scaffolds were analyzed to assess the efficacy of this process in fabricating scaffolds with different winding angles and fibre diameters. Specimens were dissected into 8 pieces where a random point on 3 replicates of each segment was imaged by light microscopy (Axio Lab A1, ZEISS) to investigate the effect of varying collector to needle distance thought the print (Figure 27). These segments were accordingly named where the ascending aorta is basically the top section of scaffold and the left ventricle is titled as the bottom of scaffold. Each sample was placed in between two microscopy glass slides to flatten the scaffold pieces and allow for more consistency across all sections. In total 81 images were taken for every independent scaffold winding angle and the images were exported to an image analysis software (ImageJ) to measure the fibre diameter and winding angles. Afterwards the resultant data was statistically analyzed and plotted using Minitab 18 to quantitatively evaluate the consistency of print across all samples while comparing the winding angle fibre diameter across different groups.

### 2.2 Results and discussion

Scaffolds were successfully fabricated with a good qualitative finishing with a constant surface thickness throughout the whole construct. Microscopic images shown in Figure 27 visibly highlight the difference in a winding angle across pre-programmed 30, 45 and 60º scaffolds as well as the sinuses for each individual scaffold. Distinct identifiable fibre winding angles, intuitively produce anisotropic properties as reported [*Biointerphases* 7*,* 1-16 (2012)] for tubular melt electrowritten scaffolds.

The illustrated qualitative analysis was corroborated with a statistical evaluation of the measured fibre winding angle across all samples and replicates for the aortic root and the sinuses as shown in Figure 31a. The winding angle of deposited melt electrowritten fibres across all scaffold groups closely resembles the pre-programmed 30, 45 and 60º configured angles calculated using equations listed in the appendix. All calculations have been made to predict and predefine the fibre winding angle across the aortic wall. As for the 30, 45 and 60º configuration, the fibres were laid down at 29.25 ± 3.08º, 45.22 ± 4.96 º and 56.55 ± 2.14º validating the efficacy of this method to predefine fibre winding angle. Since all printing parameters were kept constant throughout the melt electrowriting process, a higher winding angle was observed across the sinuses for all group of scaffolds (Figure 31b). The fibre winding angle across the sinuses were consistently 4.5 degrees larger than the measured angle on the wall for all of the configurations. One-way ANOVA and a post-hoc Tukey's multiple comparison statistical analysis was performed on all of the scaffold configurations to ensure the statistical significance of the aforementioned relationship. As it is shown in Figure 31, the winding angle across the wall and sinuses for all groups of scaffolds are significantly different according to the performed multiple comparison test. Smaller needle-to-collector distance leads to a higher effective tangential velocity on the sinuses which results into a higher winding angle of fibres at this region. In fact, the established relationship between the tangential speed and fibre winding angle allowed the prediction of the fibre winding angle across the gradient of heights apparent at the sinuses of Valsalva. These data correlates well with the mechanical properties of a native aortic valve sinuses as studies entitle the sinuses of an aortic root to be more mechanically robust compare to the aortic wall tissue.

In addition to the winding angle, fibre diameter was measured across the wall and sinuses of all three scaffold configurations. An inverse relationship between the fibre diameter and configured winding angle is clearly illustrated in Figure 31b. A higher mandrel rotational speed for a 60° scaffold induces a larger stretching effect on the deposited fibres in compared with a 30° and 45° scaffold. This phenomenon results in a drop in fibre diameter from 20.24 ± 1.59 mm for a 30º scaffold to 18.03 ± 1.38 mm and 14.84 ± 1.63 mm for a 45° and 60° scaffold, respectively. Moreover, Figure 31c confirms the influence of variable needle to collector distance on jet pulsing. Fibres deposited over the sinuses were shown to be distinctively larger in diameter by an average of 4.3 µm compared to their corresponding scaffold wall. Tukey multiple comparison statistical test authenticated statistical difference between the fibre diameter across the wall and sinuses for all three groups of scaffolds.

Lastly, hierarchical tri-layered and multi-scale scaffolds were successfully fabricated with an ideal surface finish as illustrated in Figure 28. Further mechanical and morphological characterization will be performed for tri-layered scaffold to assess the mechanical efficacy of this construct for an aortic root position.

The utilized design and manufacturing methodologies resulted in the successful fabrication of scaffolds resembling the geometrical dimensions of the aortic root. This exemplary embodiment aimed at controlling the winding angle of PCL fibres while conforming to a pre-fabricated mould (i.e. the mandrel) that replicates the geometry of the aortic valve including the sinuses of Valsalva. Mathematical relationships between the fibre winding angle and a combination of translation speed of collector and rotation speed of the mandrel were validated by fabricating melt electrowritten scaffolds on a 3D-printed conductive mould according to Equations 1-4. $Resultant vector speed = \sqrt{Translational spee {d}^{2} + Tangential spee {d}^{2}}$ $\begin{matrix}Effective Resultant vector speed \\ = \sqrt{Effective Translational spee {d}^{2} + Tangential spee {d}^{2}}\end{matrix}$ $Actual winding angle = {tan}^{- 1} \left(\frac{tangetial speed}{translational speed}\right)$ $Effective winding angle = {tan}^{- 1} \left(\frac{tangetial speed}{effective translational speed}\right)$

A higher winding angle and fibre diameter was achieved on the sinuses of Valsalva as a result of the smaller needle-to-collector distance in this area. In addition, a higher winding angle was found to reduce the fibre diameter because of the larger mandrel rotational speed used to achieve that winding angle. Anisotropic mechanical properties are expected for this tubular MEW scaffolds where a lower winding angle is hypothesized to be stiffer in the axial direction. On the other hand, the higher winding angle is expected to have more compliance circumferentially.

*Integrating the heart valve leaflet and aortic root melt electro-spun scaffolds to fabricate the whole valve conduit.*

The mechanical and morphological properties of the flat and tubular personalized scaffolds have been optimized toward the properties of an aortic heart valve leaflet and aortic root respectively. However, these scaffolds are fabricated by different collector (i.e. mandrel) setups which does not allow for the fabrication of both scaffolds in one step. In order to fabricate a scaffold for the aortic heart valve position, the flat scaffold can be integrated into the tubular aortic root scaffold while mimicking the dimensions and design of the valve. Alternatively, a multi-step design and fabrication framework can be used for the incorporation of leaflets scaffolds into the tubular aortic root scaffold (e.g. Figure 25).

The pre-established optimal flat melt electro-spun scaffold is laser cut (laser cutting device) to the dimensions of the leaflets and wrapped around the 3D-printed model. Locally heating the scaffold at the commissural points creates three fusion points conforming the scaffold into concave profile conforming to allow for the coaptation seen in the native aortic leaflet (Figure 25b). Afterwards, an aluminum mandrel collector was used to hold these two pieces of the mandrel together for the tubular MEW fabrication process. The sinuses of Valsalva act as a shielding mandrel that blocks the fibre deposition from the electrowiting step of forming the leaflet scaffold.

The tubular melt electro-spun scaffold was successfully fabricated on the 2-piece model entailing the flat leaflet scaffold in the tube. The leaflets were seamlessly attached to the inside of tubular scaffold mainly at the commissures and inter-leaflet triangle areas of the aortic root. However, the attachment points seem to be weak as it was limited only to the top layer of flat and the first layer of tubular scaffold. In an attempt to improve the fusion points, the tubular scaffold was fabricated at a higher temperature (92 °C) and rotational speed where the attachment seemed to be relatively stronger compared to the previous MEW parameters. Reinforcement techniques may be required to ensure the functionality of the aortic valve scaffold under cardiovascular conditions. Reinforcing these attachment points could either be done through the MEW fabrication process or as a post-processing step after the completion of tubular melt electrowriting

In the claims which follow and in the preceding description, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the scaffold and method.

## Claims

1. A melt electrowritten soft tissue scaffold, comprising:
a body having a first region comprising a first set of fibres and a second set of fibres, the first region being anisotropic;
wherein the first set of fibres are arranged approximately parallel relative to one another, each fibre of the first set of fibres has a serpentine arrangement forming peaks and troughs, the first set of fibres has a first Young's modulus;
wherein the second set of fibres are arranged approximately parallel relative to one another, the second set of fibres being arranged transversely relative to the first set of fibres, each fibre of the second set of fibres has a serpentine arrangement forming peaks and troughs, the second set of fibres has a second Young's modulus;
wherein in the first region, one or more fibres of the second set of fibres connect adjacent fibres from the first set of fibres; and
wherein the first Young's modulus is unequal to the second Young's modulus, wherein the Young's moduli were determined by performing uniaxial tensile tests, obtaining the stress-strain curves, and calculating the Young's moduli.

2. A scaffold as claimed in claim 1, wherein the second Young's modulus is at least double the Young's modulus of the first set of fibres; wherein optionally the first set of fibres or the second set of fibres, or both, have a Young's modulus ranges from 1kPa to 10 MPa; and wherein further optionally, the second set of fibres is 5-10 times stiffer than the first set of fibres; wherein the Young's moduli were determined by performing uniaxial tensile tests, obtaining the stress-strain curves, and calculating the Young's moduli.

3. A scaffold as claimed in claim 1 or 2, wherein a pathlength of a fibre of the first set of fibres over a predefined distance is unequal to a pathlength of a fibre of the second set of fibres over the predefined distance, and optionally, wherein increasing the pathlength of the fibre of the first set of fibres relative to the path length of the fibre of the second set of fibres increases an anisotropic ratio of the first set of fibres to the second set of fibres.

4. A scaffold as claimed in any one of claims 1 to 3, wherein each fibre of the first set of fibres is separated by a first distance, and wherein each fibre of the second set of fibres is separated by a second distance; optionally, the first distance ranges from 0.1 mm to 2.5 mm; and further optionally, the second distance is unequal to the first distance, in particular, the first distance is 1-10 times larger than the second distance.

5. A scaffold as claimed in of any one of claims 1 to 4, wherein the first and second set of fibres forms at least a first layered structure.

6. A scaffold as claimed in any one of claims 1 to 5, wherein fibres of the first set of fibres are interwoven with fibres of the second set of fibres.

7. A scaffold as claimed in any one of claims 1 to 6, wherein:
the body further comprises a second region extending from the first region, the second region supporting the first region, wherein the first and second regions are optionally integral; and
optionally, the second region is a mesh having fibres arranged in a first direction and a second direction, the first and second directions being transverse to one another.

8. A scaffold as claimed in claim 7, wherein:
the first region is semicircular, optionally comprising a plurality of semicircular regions where the vertices of adjacent semicircles are positioned proximate one another; and
the second region extends from a curved side of the first region and a straight side of the first region forms an edge of the scaffold; and
optionally, there is positioned along the curved side an intermediate region positioned at an interface of the first and second regions, the intermediate region comprising a plurality of fibres, and further optionally, the intermediate region comprises:
a first set of concentric semicircle fibres that are arranged parallel to one another; and
a second set of fibres that connect adjacent concentric semicircle fibres.

9. A scaffold of any one of claims 1 to 8, wherein the first and second sets of fibres from the first region are formed from polycaprolactone.

10. A scaffold as claimed in of any one of claims 1 to 9, wherein the first region forms part of a heart valve leaflet scaffold, wherein the first set of fibres are orientated generally in a radial direction of the heart valve leaflets and the second set of fibres are orientated generally in a circumferential direction of the heart valve leaflets.

11. A scaffold as claimed in any one of claims 1 to 10, further comprising a hydrogel, wherein at least a portion of the first region is embedded in the hydrogel.

12. A scaffold as claimed in of any one of claims 1 to 11, wherein the scaffold comprises a planar region and/or tubular region.

13. A method of producing an anisotropic soft tissue scaffold using melt electrowriting, the method comprising:
extruding a polymer melt through a nozzle to form a fibre;
depositing the fibre to form a body having a first region that is anisotropic, the first region comprising:
a first set of fibres that are arranged approximately parallel to one another, each fibre of the first set of fibres has a serpentine arrangement forming peaks and troughs; and
a second set of fibres that are arranged approximately parallel relative to one another, the second set of fibres being arranged transversely relative to the first set of fibres, each fibre of the second set of fibres having a serpentine arrangement forming peaks and troughs;
wherein in the first region, one or more fibres of the second set of fibres connect adjacent fibres from the first set of fibres; and
wherein the first set of fibres are deposited so that the first set of fibres has a first Young's modulus and the second set of fibres are deposited so that the second set of fibres has a second Young's modulus.

14. A method as claimed in claim 13, wherein:
the first and second set of fibres are deposited so that fibres of the first set of fibres are interwoven with fibres of the second set of fibres; and/or
the first and second set of fibres are deposited so that a portion of the first set of fibres is fused to a portion of the second set of fibres; and/or
wherein the first and second set of fibres are deposited so that they form a layered structure.

15. A method as claimed in claim 13 or 14, further comprising depositing the fibre to form a second region extending from the first region, the second region comprising a mesh having fibres arranged in a first direction and a second direction, the first and second directions being transverse to one another.

## Patentansprüche

1. Durch Schmelz-Elektrodruck hergestelltes Gerüst für Weichgewebe, umfassend:
ein Körper mit einem ersten Bereich, der eine erste Faserbündelgruppe und eine zweite Faserbündelgruppe umfasst, wobei der erste Bereich anisotrop ist;
wobei die erste Faserbündelgruppe annähernd parallel zueinander angeordnet ist, jede Faser der ersten Faserbündelgruppe eine schlangenförmige Anordnung aufweist, die Erhebungen und Vertiefungen bildet, und die erste Faserbündelgruppe einen ersten Elastizitätsmodul aufweist;
wobei der zweite Faserbündel zueinander annähernd parallel angeordnet ist, wobei der zweite Faserbündel quer zum ersten Faserbündel angeordnet ist, jede Faser des zweiten Faserbündels eine schlangenförmige Anordnung aufweist, die Erhebungen und Vertiefungen bildet, und der zweite Faserbündel einen zweiten Elastizitätsmodul aufweist;
wobei in dem ersten Bereich eine oder mehrere Fasern des zweiten Fasersatzes benachbarte Fasern des ersten Fasersatzes miteinander verbinden; und
wobei der erste Elastizitätsmodul nicht dem zweiten Elastizitätsmodul entspricht, wobei die Elastizitätsmodule durch die Durchführung von einachsigen Zugversuchen, die Ermittlung der Spannungs-Dehnungs-Kurven und die Berechnung der Elastizitätsmodule bestimmt wurden.

2. Gerüst nach Anspruch 1, wobei der zweite Elastizitätsmodul mindestens doppelt so groß ist wie der Elastizitätsmodul des ersten Fasersatzes; wobei optional der erste Fasersatz oder der zweite Fasersatz oder beide einen Elastizitätsmodul im Bereich von 1kPa bis 10 MPa aufweisen; und wobei ferner optional der zweite Fasersatz 5- bis 10-mal steifer ist als der erste Fasersatz; wobei die Elastizitätsmodule
ermittelt durch Durchführung von
uniaxiale Zugversuche, Ermittlung der Spannungs-Dehnungs-Kurven und Berechnung der Elastizitätsmoduli.

3. Gerüst nach Anspruch 1 oder 2, wobei eine Weglänge einer Faser des ersten Fasersatzes über eine vordefinierte Strecke ungleich einer Weglänge einer Faser des zweiten Fasersatzes über die vordefinierte Strecke ist, und gegebenenfalls **dadurch gekennzeichnet, dass** eine Vergrößerung der Weglänge der Faser des ersten Fasersatzes im Verhältnis zur Weglänge der Faser des zweiten Fasersatzes ein Anisotropieverhältnis des ersten Fasersatzes zum zweiten Fasersatz erhöht.

4. Gerüst nach einem der Ansprüche 1 bis 3, wobei jede Faser des ersten Fasersatzes durch einen ersten Abstand voneinander getrennt ist und wobei jede Faser des zweiten Fasersatzes durch einen zweiten Abstand voneinander getrennt ist; optional liegt der erste Abstand im Bereich von 0,1 mm bis 2,5 mm; und ferner optional ist der zweite Abstand ungleich dem ersten Abstand, insbesondere ist der erste Abstand 1- bis 10-mal größer als der zweite Abstand.

5. Gerüst nach einem der Ansprüche 1 bis 4, wobei der erste und der zweite Fasersatz mindestens eine erste Schichtstruktur bilden.

6. Gerüst nach einem der Ansprüche 1 bis 5, wobei Fasern des ersten Fasersatzes mit Fasern des zweiten Fasersatzes verflochten sind.

7. Gerüst nach einem der Ansprüche 1 bis 6, wobei:
der Körper umfasst ferner einen zweiten Bereich, der sich vom ersten Bereich aus erstreckt, wobei der zweite Bereich den ersten Bereich stützt, wobei der erste und der zweite Bereich gegebenenfalls einstückig ausgebildet sind; und
Optional ist der zweite Bereich ein Netz, dessen Fasern in einer ersten Richtung und einer zweiten Richtung angeordnet sind, wobei die erste und die zweite Richtung senkrecht zueinander stehen.

8. Gerüst nach Anspruch 7, wobei:
der erste Bereich ist halbkreisförmig und umfasst gegebenenfalls mehrere halbkreisförmige Bereiche, wobei die Scheitelpunkte benachbarter Halbkreise nahe beieinander liegen; und
der zweite Bereich erstreckt sich von einer gekrümmten Seite des ersten Bereichs aus, und eine gerade Seite des ersten Bereichs bildet eine Kante des Gerüsts; und
Optional ist entlang der gekrümmten Seite ein Zwischenbereich angeordnet, der an der Grenzfläche zwischen dem ersten und dem zweiten Bereich liegt, wobei der Zwischenbereich eine Vielzahl von Fasern umfasst, und ferner optional umfasst der Zwischenbereich:
eine erste Gruppe konzentrischer, halbkreisförmiger Fasern, die parallel zueinander angeordnet sind; und
ein zweiter Faserbündel, der benachbarte konzentrische Halbkreisfasern miteinander verbindet.

9. Gerüst gemäß einem der Ansprüche 1 bis 8, wobei der erste und der zweite Fasersatz aus dem ersten Bereich aus Polycaprolacton bestehen.

10. Gerüst gemäß einem der Ansprüche 1 bis 9, wobei der erste Bereich einen Teil eines Herzklappensegelgerüsts bildet, wobei der erste Fasersatz im Wesentlichen in radialer Richtung der Herzklappensegel ausgerichtet ist und der zweite Fasersatz im Wesentlichen in Umfangsrichtung der Herzklappensegel ausgerichtet ist.

11. Gerüst nach einem der Ansprüche 1 bis 10, das ferner ein Hydrogel umfasst, wobei mindestens ein Teil des ersten Bereichs in das Hydrogel eingebettet ist.

12. Gerüst nach einem der Ansprüche 1 bis 11, wobei das Gerüst einen ebenen Bereich und/oder einen röhrenförmigen Bereich umfasst.

13. Verfahren zur Herstellung eines anisotropen Weichgewebe-Gerüsts mittels Schmelz-Elektroschreiben, wobei das Verfahren umfasst:
das Extrudieren einer Polymerschmelze durch eine Düse zur Bildung einer Faser;
Aufbringen der Faser zur Bildung eines Körpers mit einem ersten Bereich, der anisotrop ist, wobei der erste Bereich Folgendes umfasst:
eine erste Gruppe von Fasern, die annähernd parallel zueinander angeordnet sind, wobei jede Faser der ersten Gruppe eine schlangenförmige Anordnung aufweist, die Erhebungen und Vertiefungen bildet; und
eine zweite Gruppe von Fasern, die im Wesentlichen parallel zueinander angeordnet sind, wobei die zweite Gruppe von Fasern quer zur ersten Gruppe von Fasern verläuft und jede Faser der zweiten Gruppe von Fasern eine wellenförmige Anordnung aufweist, die Erhebungen und Vertiefungen bildet;
wobei in dem ersten Bereich eine oder mehrere Fasern des zweiten Fasersatzes benachbarte Fasern des ersten Fasersatzes miteinander verbinden; und
wobei der erste Fasersatz so aufgebracht wird, dass er einen ersten Elastizitätsmodul aufweist, und der zweite Fasersatz so aufgebracht wird, dass er einen zweiten Elastizitätsmodul aufweist.

14. Verfahren nach Anspruch 13, wobei:
der erste und der zweite Fasersatz werden so aufgebracht, dass Fasern des ersten Fasersatzes mit Fasern des zweiten Fasersatzes verflochten sind; und/oder
die erste und die zweite Faserbahn werden so aufgebracht, dass ein Teil der ersten Faserbahn mit einem Teil der zweiten Faserbahn verschmilzt; und/oder
wobei der erste und der zweite Faserbündel so aufgebracht werden, dass sie eine Schichtstruktur bilden.

15. Verfahren nach Anspruch 13 oder 14, das ferner das Aufbringen der Faser umfasst, um einen zweiten Bereich zu bilden, der sich vom ersten Bereich aus erstreckt, wobei der zweite Bereich ein Gewebe umfasst, dessen Fasern in einer ersten Richtung und einer zweiten Richtung angeordnet sind, wobei die erste und die zweite Richtung senkrecht zueinander stehen.

## Revendications

1. Échafaudage de tissu mou par électroimpression à chaud, comprenant:
un corps comportant une première région constituée d'un premier ensemble de fibres et d'un deuxième ensemble de fibres, la première région étant anisotrope;
dans lequel le premier ensemble de fibres est disposé de manière approximativement parallèle les unes par rapport aux autres, chaque fibre du premier ensemble de fibres présente une configuration sinueuse formant des crêtes et des creux, et le premier ensemble de fibres présente un premier module d'Young;
dans lequel le deuxième ensemble de fibres est disposé de manière approximativement parallèle les unes par rapport aux autres, ce deuxième ensemble de fibres étant disposé transversalement par rapport au premier ensemble de fibres, chaque fibre du deuxième ensemble de fibres présentant une disposition sinueuse formant des crêtes et des creux, et le deuxième ensemble de fibres présentant un deuxième module d'Young;
dans laquelle, dans la première région, une ou plusieurs fibres du deuxième ensemble de fibres relient des fibres adjacentes du premier ensemble de fibres; et
dans lequel le premier module d'Young est différent du second module d'Young, les modules d'Young ayant été déterminés en réalisant des essais de traction uniaxiaux, en obtenant les courbes contrainte-déformation et en calculant les modules d'Young.

2. Échafaudage selon la revendication 1, dans lequel le deuxième module d'Young est au moins le double du module d'Young du premier ensemble de fibres; dans laquelle, éventuellement, le premier ensemble de fibres ou le deuxième ensemble de fibres, ou les deux, présentent un module d'Young compris entre 1kPa et 10 MPa; et dans lequel, en outre, éventuellement, le deuxième ensemble de fibres est 5 à 10 fois plus rigide que le premier ensemble de fibres; dans lequel les modules de Young ont été
déterminés en effectuant
des essais de traction uniaxiaux, permettant d'obtenir les courbes contrainte-déformation et de calculer les modules de Young.

3. Échafaudage selon la revendication 1 ou 2, dans lequel la longueur de trajet d'une fibre du premier ensemble de fibres sur une distance prédéfinie est différente de la longueur de trajet d'une fibre du deuxième ensemble de fibres sur ladite distance prédéfinie, et, éventuellement, dans lequel l'augmentation de la longueur de trajet de la fibre du premier ensemble de fibres par rapport à la longueur de trajet de la fibre du deuxième ensemble de fibres augmente le rapport d'anisotropie entre le premier ensemble de fibres et le deuxième ensemble de fibres.

4. Échafaudage selon l'une quelconque des revendications 1 à 3, dans lequel chaque fibre du premier ensemble de fibres est espacée d'une première distance, et dans laquelle chaque fibre du deuxième ensemble de fibres est espacée d'une deuxième distance; éventuellement, la première distance est comprise entre 0,1 mm et 2,5 mm; et éventuellement, la seconde distance est différente de la première distance, en particulier, la première distance est 1 à 10 fois plus grande que la seconde distance.

5. Échafaudage selon l'une quelconque des revendications 1 à 4, dans lequel les premier et deuxième ensembles de fibres forment au moins une première structure en couches.

6. Échafaudage selon l'une quelconque des revendications 1 à 5, dans lequel les fibres du premier ensemble de fibres sont entrelacées avec les fibres du deuxième ensemble de fibres.

7. Échafaudage selon l'une quelconque des revendications 1 à 6, **caractérisé en ce que**:
le corps comprend en outre une deuxième zone s'étendant à partir de la première zone, la deuxième zone servant de support à la première zone, les première et deuxième zones pouvant être d'un seul tenant; et
Le cas échéant, la deuxième région est un maillage dont les fibres sont disposées selon une première direction et une deuxième direction, ces deux directions étant transversales l'une par rapport à l'autre.

8. Échafaudage selon la revendication 7, **caractérisé en ce que**:
la première région est semi-circulaire, pouvant éventuellement comprendre une pluralité de régions semi-circulaires dont les sommets des demi-cercles adjacents sont situés à proximité les uns des autres; et
la deuxième région s'étend à partir d'un côté courbe de la première région, tandis qu'un côté droit de cette dernière forme un bord de l'échafaudage; et
en option, une zone intermédiaire est disposée le long du côté courbe, à l'interface entre la première et la deuxième zone; cette zone intermédiaire comprend une pluralité de fibres; et, en option également, la zone intermédiaire comprend:
un premier ensemble de fibres en demi-cercles concentriques disposées parallèlement les unes aux autres; et
un deuxième ensemble de fibres reliant des fibres semi-circulaires concentriques adjacentes.

9. Échafaudage selon l'une quelconque des revendications 1 à 8, dans lequel les premier et deuxième ensembles de fibres provenant de la première région sont constitués de polycaprolactone.

10. Échafaudage selon l'une quelconque des revendications 1 à 9, dans lequel la première région fait partie d'une structure de valve cardiaque, le premier ensemble de fibres étant orienté globalement dans une direction radiale par rapport aux valves cardiaques et le deuxième ensemble de fibres étant orienté globalement dans une direction circonférentielle par rapport aux valves cardiaques.

11. Échafaudage selon l'une quelconque des revendications 1 à 10, comprenant en outre un hydrogel, dans lequel au moins une partie de la première région est noyée dans l'hydrogel.

12. Échafaudage selon l'une quelconque des revendications 1 à 11, **caractérisé en ce que** ledit échafaudage comprend une région plane et/ou une région tubulaire.

13. Procédé de fabrication d'un support anisotrope pour tissus mous par électro-écriture à l'état fondu, ledit procédé comprenant:
extruder une masse fondue de polymère à travers une buse pour former une fibre;
dépôt de la fibre pour former un corps comportant une première région anisotrope, cette première région comprenant:
un premier ensemble de fibres disposées de manière approximativement parallèle les unes aux autres, chaque fibre de ce premier ensemble présentant une configuration sinueuse formant des crêtes et des creux; et
un deuxième ensemble de fibres disposées de manière approximativement parallèle les unes par rapport aux autres, ce deuxième ensemble de fibres étant disposé transversalement par rapport au premier ensemble de fibres, chaque fibre du deuxième ensemble de fibres présentant une disposition en serpentine formant des crêtes et des creux;
dans laquelle, dans la première région, une ou plusieurs fibres du deuxième ensemble de fibres relient des fibres adjacentes du premier ensemble de fibres; et
dans lequel le premier ensemble de fibres est déposé de telle sorte que ce premier ensemble de fibres présente un premier module d'Young, et le deuxième ensemble de fibres est déposé de telle sorte que ce deuxième ensemble de fibres présente un deuxième module d'Young.

14. Procédé selon la revendication 13, **caractérisé en ce que**:
le premier et le deuxième ensemble de fibres sont déposés de telle sorte que les fibres du premier ensemble s'entrelacent avec celles du deuxième ensemble; et/ou
les premier et deuxième ensembles de fibres sont déposés de telle sorte qu'une partie du premier ensemble de fibres soit fusionnée à une partie du deuxième ensemble de fibres; et/ou
dans laquelle les premier et deuxième ensembles de fibres sont déposés de manière à former une structure en couches.

15. Procédé selon la revendication 13 ou 14, comprenant en outre le dépôt de la fibre pour former une deuxième région s'étendant à partir de la première région, la deuxième région comprenant un maillage dont les fibres sont disposées selon une première direction et une deuxième direction, la première et la deuxième directions étant transversales l'une par rapport à l'autre.
